(19) **Europäisches Patentamt / European Patent Office / Office européen des brevets**

(11) **EP 4 346 557 B1**

(12) **FASCICULE DE BREVET EUROPEEN**

(45) Date de publication et mention
de la délivrance du brevet:
**21.05.2025 Bulletin 2025/21**

(21) Numéro de dépôt: **22731196.6**

(22) Date de dépôt: **31.05.2022**

(51) Classification Internationale des Brevets (IPC):
**A61B 5/00** (2006.01)   **A61B 5/0531** (2021.01)
**A61B 5/145** (2006.01)   **G16H 10/40** (2018.01)
**G16H 40/63** (2018.01)   **G16H 50/20** (2018.01)

(52) Classification Coopérative des Brevets (CPC):
**A61B 5/14517; G16H 10/40; G16H 50/20**

(86) Numéro de dépôt international:
**PCT/EP2022/064783**

(87) Numéro de publication internationale:
**WO 2022/253843 (08.12.2022 Gazette 2022/49)**

(54) **DETECTION D'UNE ESPECE CHIMIQUE DANS LA SUEUR D'UN SUJET**

NACHWEIS EINER CHEMISCHEN SPEZIES IM SCHWEISS EINER PERSON

DETECTION OF A CHEMICAL SPECIES IN THE SWEAT OF A SUBJECT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats de validation désignés:
**MA TN**

(30) Priorité: **02.06.2021 FR 2105827**

(43) Date de publication de la demande:
**10.04.2024 Bulletin 2024/15**

(73) Titulaire: **NOPTRACK
81860 Castres (FR)**

(72) Inventeurs:
• **AMATORE, Christian
75013 Paris (FR)**
• **FAVRE, Gilles
31270 Cugnaux (FR)**
• **BEDIOUI, Fethi
75010 Paris (FR)**
• **CHEN, Yong
75005 Paris (FR)**
• **GRIVEAU, Sophie
91300 Massy (FR)**
• **SELLA, Catherine
92360 Meudon la forêt (FR)**
• **THOUIN, Laurent
92160 Antony (FR)**

(74) Mandataire: **Loyer & Abello
9, rue Anatole de la Forge
75017 Paris (FR)**

(56) Documents cités:
**WO-A1-2019/229380      WO-A2-2016/061362
US-A1- 2019 110 722**

• **KENNY WALTER: "Novel Wearable Sensor
Monitors Health Through Sweat Using
Nanotech", -RESEARCH & DEVELOPMENT
WORLD, 26 April 2019 (2019-04-26),
XP055738166, Retrieved from the Internet
<URL:https://www.rdworldonline.com/
novel-wearable-sensor-monitors-health-
through-sweat-using-nanotech/> [retrieved on
20201008]**
• **JUNGIL CHOI ET AL: "Skin-interfaced systems
for sweat collection and analytics", SCIENCE,
vol. 4, no. 2, 16 February 2018 (2018-02-16), US,
pages eaar3921, XP055526287, ISSN: 0036-8075,
DOI: 10.1126/sciadv.aar3921**
• **YEJIN HA ET AL: "Measurements of Location-
Dependent Nitric Oxide Levels on Skin Surface in
relation to Acupuncture Point", EVIDENCE-
BASED COMPLEMENTARY AND ALTERNATIVE
MEDICINE, vol. 2012, 1 January 2012
(2012-01-01), US, pages 1 - 7, XP055524807,
ISSN: 1741-427X, DOI: 10.1155/2012/781460**

- **RICHARD WELLER ET AL: "Nitric Oxide Is Generated on the Skin Surface by Reduction of Sweat Nitrate", JOURNAL OF INVESTIGATIVE DERMATOLOGY, vol. 107, no. 3, 1 September 1996 (1996-09-01), pages 327 - 331, XP055205544, DOI: 10.1111/ 1523-1747.ep12363167**
- **YOKOTA K ET AL: "MOLECULAR CLONING OF A NEW HUMAN C-TYPE LECTIN WITH SIGNIFICANT HOMOLOGY TO MOUSE DECTIN-2", JOURNAL OF INVESTIGATIVE DERMATOLOGY, ELSEVIER, NL, vol. 110, no. 4, 1 April 1998 (1998-04-01), pages 1, XP000828222, ISSN: 0022-202X, DOI: 10.1046/ J.1523-1747.1998.00084.X**
- **BYEONG WAN AN ET AL: "Smart Sensor Systems for Wearable Electronic Devices", POLYMERS, vol. 9, no. 12, 25 July 2017 (2017-07-25), pages 303, XP055526164, DOI: 10.3390/polym9080303**
- **ANASTASOVA SALZITSA ET AL: "A wearable multisensing patch for continuous sweat monitoring", vol. 93, 1 July 2017 (2017-07-01), Amsterdam , NL, pages 139 - 145, XP055818838, ISSN: 0956-5663, Retrieved from the Internet <URL:https://www.researchgate.net/profile/ Salzitsa-Anastasova/publication/308491565_A_ Wearable_Multisensing_Patch_for_Continuous_ Sweat_Monitoring/links/ 59461f8caca2722db4a32b81/ A-Wearable-Multisensing-Patch-for-Conti nuous-Sweat-Monitoring.pdf> DOI: 10.1016/ j.bios.2016.09.038**

- **SHERIDAN EOIN ET AL: "Bipolar electrode depletion: membraneless filtration of charged species using an electrogenerated electric field gradient", ANALYST, vol. 136, no. 20, 1 January 2011 (2011-01-01), UK, pages 4134, XP055963569, ISSN: 0003-2654, Retrieved from the Internet <URL:https://pubs.rsc.org/en/content/articlepdf/ 2011/an/c1an15510e> DOI: 10.1039/c1an15510e**

## Description

### Domaine technique

L'invention concerne des procédés et appareils pour détecter et mesurer des espèces chimiques dissoutes dans la sueur d'un sujet humain ou animal, en particulier le monoxyde d'azote.

### Arrière-plan technologique

Le monoxyde d'azote est un gaz qui constitue un messager intercellulaire. Il est un important messager cardiovasculaire du stress par mécanotransduction. Il est émis en particulier afin de stimuler la vasodilatation du système vasculaire lors d'efforts musculaires. Les variations de son flux de production dans le sang et donc dans les liquides en équilibre avec le sang, par exemple la sueur, constituent donc un indicateur particulièrement pertinent de la capacité cardiovasculaire d'un patient à s'adapter à la puissance musculaire demandée lors de tests d'effort.

Ainsi, il existe un réel besoin de développer un appareil de détection de monoxyde d'azote (NO) facile à fabriquer, fiable et facile d'utilisation.

Dans le document WO2019229380A1 on a décrit un appareil permettant une mesure instantanée du monoxyde d'azote dans la sueur au niveau de l'épiderme d'un sujet et ses applications cliniques.

Le document US 2019/110722 A1 divulgue un appareil de détection de biomarqueurs pouvant comprendre le peroxyde d'hydrogène. En outre, US 2019/110722 A1 divulgue une liste d'exemples de biomarqueurs qui peuvent être trouvés dans la sueur et utilisés dans des applications de surveillance de la santé (para [0092]) dont le NO ne fait pas parti. La publication Kenny Walter, in Research & Development World, du 26 avril 2019 divulgue un appareil permettant la détection du peroxyde d'hydrogène. La publication JUNGIL CHOI ET AL. du 16 février 2018 s'intéresse à l'origine du monoxyde d'azote (NO) apparemment émis par la surface de la peau (page 327, droite). Les seules mesures quantitatives de NO mentionnées dans ce document sont décrites dans le paragraphe intitulé NO. Il est ainsi décrit que le NO a été détecté en phase gazeuse par un analyseur de NO commercial par chimiluminescence après entraînement par un flux d'air purifié à débit contrôlé balayant la zone de la peau testée isolée dans un manchon scellé. Le document WO 2016/061362 A2 enseigne une pluralité de structures microfluidiques 230, mais ne divulgue pas un capteur électrochimique réalisant deux opérations dans le même canal microfluidique tel que revendiqué. La publication SHERIDAN FOIN ET AL. du 1 janvier 2011 concerne un dispositif de traitement des fluides à très faible conductivité, notamment pour la désalinisation des fluides, mais ne concerne pas la détection de NO.

### Résumé

Certains aspects de l'invention sont basés sur l'idée que la mesure quantitative des variations de la concentration de monoxyde d'azote dans la sueur fournit une méthode non invasive de surveillance de la capacité cardiovasculaire lors de contrôles préventifs ou afin d'établir un diagnostic.

Certains aspects de l'invention sont basés sur le constat que, en présence de dioxygène, le monoxyde d'azote réagit spontanément pour produire un ion nitrite ($NO_2^-$) via une réaction de stœchiométrie globale 1:1. En d'autres termes, tandis que les variations de la concentration de monoxyde d'azote représentent l'état présent des réponses du système cardiovasculaire à un effort donné, les variations de la concentration de l'ion nitrite constituent une archive temporelle de ces réponses.

Certains aspects de l'invention sont basés sur l'idée de détecter une pluralité de composés chimiques de manière couplée par un dispositif électrochimique intégré.

Certains aspects de l'invention sont basés sur l'idée que les variations de concentrations du monoxyde d'azote et de l'ion nitrite peuvent être détectées et quantifiées de manière couplée par un dispositif électrochimique intégré.

Certains aspects de l'invention sont basés sur le constat que le monoxyde d'azote est produit par des enzymes spécialisées (les NO-synthases) à partir de la dégradation de L-arginine intracellulaire en présence de dioxygène ($O_2$) et d'une source d'électrons. Lorsque la disponibilité en L-arginine diminue du fait d'une forte consommation (par exemple à la suite d'un effort prolongé) ou d'une carence chronique, les NO-synthases continuent à réagir avec l'oxygène en se bornant à réduire le dioxygène en ion su-peroxyde ($O_2^-$). Ce dernier évolue spontanément très rapidement en peroxyde d'hydrogène ($H_2O_2$), via une réaction de stœchiométrie globale 2:1. Certains aspects de l'invention sont basés sur l'idée que la présence de concentrations détectables de peroxyde d'hydrogène dans le sang, et donc dans la sueur, fournit un indicateur représentant un niveau de souffrance du réseau cardiovasculaire. De plus, en présence de sels métalliques, peroxyde d'hydrogène se décompose en espèces radicalaires très toxiques ($HO\bullet$, $HO_2\bullet$, etc.) susceptibles d'induire des dégâts très importants sur les cellules du système cardiovasculaire y compris celles du cœur. Certains aspects de l'invention sont basés sur l'idée que la détection d'une production de peroxyde d'hydrogène en parallèle de la détection d'une production de monoxyde d'azote et/ou d'ion nitrite est pertinente pour évaluer les capacités cardiovasculaires d'un patient.

Certains aspects de l'invention sont basés sur l'idée de mesurer la quantité au moins du monoxyde d'azote par un dispositif électrochimique et de l'ion nitrite par une technique de colorimétrie.

Certains aspects de l'invention sont basés sur le constat que le système physiologique d'un sujet est dynamique, puisque le débit volumique de sueur peut varier pour ajuster la capacité d'élimination des calories produites en fonction de la puissance musculaire délivrée. Les flux d'échange de chaque espèce chimique au niveau des interfaces entre le sang et la sueur peuvent varier en fonction d'un effort fourni par le sujet. Certains aspects de l'invention sont basés sur l'idée de détecter de manière quantitative et dynamique la production d'une ou plusieurs des espèces chimiques choisies parmi le monoxyde d'azote, l'ion nitrite, le peroxyde d'hydrogène et éventuellement le peroxynitrite, par exemple lors d'un test d'effort ou d'un suivi médical du sujet.

Pour cela, l'invention fournit un appareil de détection destiné à être posé sur une zone d'investigation d'un épiderme d'un sujet humain ou animal pour détecter au moins le monoxyde d'azote dissout dans la sueur, ledit appareil de détection comportant :

une structure définissant un circuit microfluidique pour conduire un flux de sueur, la structure comprenant un orifice d'entrée laissant passer la sueur depuis l'épiderme, le circuit microfluidique comportant au moins un canal microfluidique en communication avec l'orifice d'entrée,

au moins un capteur électrochimique comportant au moins quatre électrodes disposées de manière successive dans une direction longitudinale du canal microfluidique, les au moins quatre électrodes comportant une électrode de référence, au moins deux électrodes de travail et une contre-électrode,

le capteur électrochimique étant configuré pour produire au moins un signal représentatif d'une concentration du monoxyde d'azote dissout dans le flux de sueur et configuré en outre pour faire au moins une opération supplémentaire parmi :

réaliser une déplétion d'une espèce chimique dans le flux de sueur, ladite espèce chimique présentant un potentiel d'oxydation inférieur au potentiel d'oxydation du monoxyde d'azote et

produire un signal représentatif d'une vitesse d'écoulement du flux de sueur.

Grâce à ces caractéristiques, il est possible de mesurer la concentration de monoxyde d'azote (NO) dissout dans la sueur de manière fiable.

On entend par un épiderme la couche superficielle de la peau chez l'homme et les animaux.

L'appareil de détection est non invasif et ne nécessite pas d'être appliqué sur une plaie.

Selon des modes de réalisation, un tel appareil peut comporter une ou plusieurs des caractéristiques suivantes.

Selon un mode de réalisation, la structure est une structure multicouche comportant une couche inférieure et au moins une couche superposée sur la couche inférieure, le circuit microfluidique s'étendant parallèlement à la couche inférieure, la couche inférieure comprenant ledit orifice d'entrée.

Selon un mode de réalisation, le ou chaque ou au moins un dit capteur électrochimique est configuré pour produire un signal représentatif de la vitesse d'écoulement du flux de sueur dans le canal microfluidique, et donc d'un débit volumique de sueur dans le canal microfluidique.

Selon un mode de réalisation, la structure multicouche comporte une couche supérieure et au moins une couche intermédiaire située entre la couche inférieure et la couche supérieure, le circuit microfluidique étant formé dans l'épaisseur de la au moins une couche intermédiaire. Les couches peuvent être fixées les unes aux autres par toute méthode appropriée, par exemple par des adhésifs, par soudage, par serrage mécanique etc.

Grâce à ces caractéristiques, la fabrication, le montage et donc l'industrialisation de l'appareil de détection est facilité.

Selon un mode de réalisation, la au moins une couche intermédiaire comporte une première couche intermédiaire et une deuxième couche intermédiaire d'étanchéité située entre la première couche intermédiaire et la couche supérieure, la deuxième couche intermédiaire d'étanchéité comportant une ouverture au niveau des électrodes.

Grâce à ces caractéristiques, l'appareil de détection est adapté aux éventuelles courbures lorsqu'il est appliqué sur l'épiderme. En outre, la ou les couches intermédiaires permettent également de créer une épaisseur permettant de compenser l'épaisseur des au moins quatre électrodes. Ainsi, l'étanchéité de l'appareil de détection est assurée.

Selon un mode de réalisation, la au moins une couche intermédiaire comporte un orifice de sortie permettant d'évacuer le flux de sueur ayant subi la ou les mesures électrochimiques.

Selon un mode de réalisation, la structure multicouche comporte une couche supérieure et un orifice de sortie traversant la couche supérieure, dans lequel le au moins un canal microfluidique est en communication avec l'orifice de sortie.

Selon un mode de réalisation, la couche inférieure est revêtue d'un adhésif fabriqué en matériau souple biocompatible. Selon un mode de réalisation, la couche inférieure est adhésive sur une première face destinée à être positionnée sur la peau et une seconde face destiné à recevoir la ou les couches superposées.

Grâce à ces caractéristiques, l'appareil de détection mesure une concentration d'au moins la concentration du monoxyde d'azote sans perturber la circulation de la sueur au niveau de la zone d'investigation de l'épiderme du sujet. C'est-à-dire que l'appareil de détection fonctionne sans altérer le passage de la sueur à travers l'épiderme au niveau de la zone d'investigation de l'épiderme du sujet.

Selon un mode de réalisation, la structure multicouche est fabriquée à partir d'un ou plusieurs matériaux suivants :

- Inorganique tel que la silice, le verre, le verre photosensible ;
- Elastomère tel que le polydiméthylsiloxane (PDMS), le polydiméthylsiloxane (PDMS) modifié avec (acide polyacrylique, poly(oxyde d'éthylène), TiO2, film d'aluminium, polycations, polyanion, mPEG, sol-gel, amine -thiol-carboxyl al-koxysianes, surfactants), le poly(méthylméthacrylate) (PMMA), le polycarbonate (PC) ;
- Thermodurcissable tel que la résine photosensible SU-8 ;
- Thermoplastique tel que le polystyrène (PS), le téréphtalate de polyéthylène (PET), le chlorure de polyvinyle (PVC), le perfluoroalkoxy (Teflon-PFA ®), l'éthylène-propylène fluoré (Téflon-FEP ®) ;
- Hydrogel tel que le matrigel ®, le collagène, le chitosan, l'alginate, l'agarose, PEG et la polyacrylamide ;
- Papier tel que la cellulose.

Selon un mode de réalisation, la structure multicouche comporte au moins une couche fabriquée en matériaux polymères. Par exemple, pour tracer le circuit microfluidique dans un matériau polymère, on peut utiliser des techniques de découpe de membranes ou de moulage.

Selon un mode de réalisation, la structure multicouche est fabriquée en matériau polymère.

Selon un mode de réalisation, la structure multicouche comporte au moins une couche fabriquée en matériaux fibreux, par exemple papier ou intissé. Par exemple, pour tracer le circuit microfluidique dans un matériau fibreux, on peut utiliser un revêtement d'encre hydrophobe ou de résine hydrophobe délimitant les contours du circuit microfluidique.

Grâce à ces caractéristiques, la structure multicouche est légère, souple et flexible. Cela permet notamment de permettre l'utilisation de l'appareil de détection sur de nombreuses parties du corps tel que le dos, le bras, l'épaule, la jambe ou la nuque sans risquer de l'endommager ou de diminuer la fiabilité des détections.

Selon un mode de réalisation, la structure multicouche comporte au moins une couche fabriquée en verre.

De manière préférée, la structure multicouche est fabriquée à partir de : Polyéthylène téréphtalate (PET), Naphtalate de polyéthylène (PEN), Adhésif acrylique, polybutyrate adipate téréphtalate (PBAT), Couche de polyuréthane et couche de polyacrylate (MPU).

Selon un mode de réalisation, les au moins quatre électrodes sont disposées sur une face interne de la couche supérieure fermant le canal microfluidique par le haut et/ou sur une face supérieure de la couche inférieure fermant le canal microfluidique par le bas.

Grâce à ces caractéristiques, les électrodes de l'appareil détecteur sont disposées de manière fiable. En outre, la fabrication de la structure multicouche comportant ces électrodes est facilitée en ce qu'il est possible de fabriquer les électrodes sur une couche plane lorsque le circuit microfluidique est formé dans une couche intermédiaire.

Selon un mode de réalisation, les électrodes sont constituées de dépôts métalliques.

Selon un mode de réalisation, les dépôts métalliques sont sélectionnés dans le groupe consistant en 1'argent (Ag), l'or (Au), le platine (Pt), et le noir de platine. On peut aussi utiliser le graphite ou le carbone. Selon un mode de réalisation, le capteur électrochimique comporte une électrode de référence argent/chlorure d'argent (Ag/AgCl) ou autre.

Selon un mode de réalisation, le capteur électrochimique est configuré pour réaliser une déplétion d'une espèce chimique présentant un potentiel d'oxydation inférieur au potentiel d'oxydation du monoxyde d'azote. Pour cela, dans un mode de réalisation, dans la direction du flux, les au moins quatre électrodes comportent successivement la première électrode de travail réalisée sous la forme d'une électrode de déplétion, la deuxième électrode de travail pour mesurer la concentration du monoxyde d'azote et la contre-électrode, l'électrode de référence étant placée à une position immédiatement en amont de la première électrode de travail ou immédiatement en aval de la deuxième électrode de travail.

Grâce à ces caractéristiques, il est possible d'accroitre la précision du capteur électrochimique, en diminuant fortement les signaux parasites pouvant être dus à des composés chimiques s'oxydant à des potentiels électriques inférieurs au potentiel électrique d'oxydation du monoxyde d'azote. Ainsi, l'ensemble des électrodes agissent en synergie afin d'obtenir un résultat précis.

Selon un mode de réalisation, l'électrode de déplétion est plus large que la deuxième électrode de travail requise pour mesurer la concentration du monoxyde d'azote, par exemple au moins quatre fois plus large.

Grâce à la grande surface de l'électrode de déplétion, l'électrolyse du flux de sueur passant au droit de l'électrode de déplétion peut être sensiblement complète. De plus, le phénomène de passivation de l'électrode est diminué.

Selon un mode de réalisation, une ou plusieurs des électrodes, par exemple l'électrode de déplétion, est recouverte de noir de platine (Pt).

Grâce à ces caractéristiques, l'électrode permet de catalyser les réactions électrochimiques par la nature imparfaite donc réactive des dendrites de noir de Pt et d'empêcher la désactivation des surfaces de métal nu.

Selon un mode de réalisation, le capteur électrochimique est configuré pour polariser l'électrode de déplétion à un potentiel électrique permettant d'oxyder au moins une espèce chimique choisie parmi : le peroxyde d'hydrogène ($H2O2$), le peroxynitrite ($ONOO^-$) et éventuellement d'autres espèces chimiques qui s'oxydent à ces potentiels.

Selon un mode de réalisation, la contre électrode du capteur électrochimique présente une largeur au moins égale à l'addition de la largeur de l'ensemble des électrodes disposées en amont de la contre-électrode.

Selon un mode de réalisation, le capteur électrochimique est configuré pour mesurer une vitesse d'écoulement du flux de sueur dans le canal microfluidique. Pour cela, selon un mode de réalisation, dans la direction du flux, les au moins quatre électrodes comportent successivement la première électrode de travail pour mesurer la concentration du monoxyde d'azote, la deuxième électrode de travail pour mesurer la concentration du monoxyde d'azote et la contre-électrode, l'électrode de référence étant placée à une position immédiatement en amont de la première électrode de travail ou immédiatement en aval de la deuxième électrode de travail.

Grâce à ces caractéristiques, il est possible de mesurer le débit du flux de sueur entre la première électrode de travail et la deuxième électrode de travail.

Selon un mode de réalisation, le capteur électrochimique est configuré pour produire le signal représentatif de la vitesse d'écoulement par mesure d'un retard entre une variation de courant dans la première électrode de travail et une variation de courant dans la deuxième électrode de travail.

Selon un mode de réalisation, la distance séparant l'électrode de travail amont et l'électrode de travail aval est de préférence inférieure à la distance parcourue par le flux en une minute. Grâce à ces caractéristiques, il est possible d'obtenir une mesure de débit précise sans perturbation pouvant être due à un changement physiologique du sujet.

Selon un mode de réalisation, le capteur électrochimique est configuré pour produire un signal représentatif d'une production instantanée du monoxyde d'azote dans la zone d'investigation sur la base du signal représentatif de la concentration du monoxyde d'azote et du signal représentatif de la vitesse d'écoulement du flux de sueur.

Par « production instantanée », on désigne une mesure prise sur une durée très faible par rapport au temps caractéristique de la variation de réponse physiologique du sujet. Ce temps caractéristique est typiquement de l'ordre de une à plusieurs minutes pour un sujet humain.

Selon un mode de réalisation, le capteur électrochimique est configuré pour produire le signal représentatif de la concentration du monoxyde d'azote par une mesure électrique, notamment ampérométrique, entre au moins une desdites électrodes de travail et la contre-électrode.

Selon un mode de réalisation, le capteur électrochimique est configuré pour polariser au moins une desdites électrodes de travail à un potentiel électrique d'oxydation du monoxyde d'azote.

Selon un mode de réalisation, le capteur électrochimique est configuré pour mesurer les concentrations d'une ou plusieurs autres espèces chimiques dans le flux de sueur en complément de la mesure de la concentration du monoxyde d'azote. Pour cela, le capteur électrochimique est configuré pour produire le signal représentatif de la concentration de la ou des espèces chimiques d'intérêts par une mesure ampérométrique.

Selon un mode de réalisation, le capteur électrochimique est configuré en outre pour produire un signal représentatif d'une concentration dans le flux de sueur d'au moins un des composés chimiques suivants : ion nitrite, peroxyde d'hydrogène et peroxynitrite, dissout dans la sueur.

Pour cela, selon un mode de réalisation, le capteur électrochimique comprend une troisième électrode de travail entre la première ou deuxième électrode de travail et la contre électrode pour faire une mesure du composé chimique.

Selon un mode de réalisation, le capteur électrochimique est configuré pour mesurer la concentration dans le flux de sueur de plusieurs composés chimiques en série dans l'ordre croissant des potentiels électriques d'oxydation des composés chimiques.

Selon un mode de réalisation, l'appareil de détection comporte en outre un dispositif de détection par colorimétrie couplé au circuit microfluidique.

Selon un mode de réalisation, un dispositif de détection par colorimétrie est relié au canal en aval du capteur électrochimique, le dispositif de détection par colorimétrie comprenant un corps poreux hydrophile imprégné d'un réactif chimique capable de réagir avec un des composés chimiques suivants : ion nitrite, peroxyde d'hydrogène, peroxynitrite, dioxyde de soufre, sulfure d'hydrogène, monoxyde d'azote, monoxyde de carbone et acide hypochloreux, dissout dans la sueur, afin de fournir un indicateur coloré indiquant une quantité dudit composé chimiques dans le flux de sueur.

Selon un mode de réalisation, le corps poreux hydrophile est choisi parmi : membrane microporeuse, papier, tissu, ouate de cellulose, intissé etc.

Selon un mode de réalisation, le réactif chimique comprend un réactif de Griess capable de réagir avec l'ion nitrite dissout dans le flux de sueur.

Selon un mode de réalisation, le dispositif de détection par colorimétrie est disposé dans l'orifice de sortie.

Selon un mode de réalisation, le circuit microfluidique comporte une pluralité de canaux microfluidiques reliés en dérivation les uns des autres à l'orifice d'entrée.

Selon des modes de réalisation décrits ci-dessous, l'appareil de détection est réalisé de manière à pouvoir détecter simultanément ou séquentiellement plusieurs, par exemple deux, trois, quatre, ou cinq espèces chimiques, incluant le monoxyde d'azote, avec un ou plusieurs capteurs électrochimiques. Selon un mode de réalisation, l'appareil de détection est réalisé de manière à pouvoir détecter la concentration des espèces chimiques choisies parmi le monoxyde d'azote NO et l'ion nitrite $NO_2$ et le peroxyde d'hydrogène $H_2O_2$.

Selon ce mode de réalisation, l'appareil de détection comporte trois canaux microfluidiques parallèles alimentés en parallèle par le même orifice d'entrée.

Selon un mode de réalisation, la couche inférieure comporte une pluralité d'orifices d'entrée et le circuit microfluidique comporte une pluralité de canaux microfluidiques indépendants reliés respectivement à chaque orifice d'entrée.

Selon un mode de réalisation, l'appareil de détection comporte un corps fibreux pour conduire la sueur de la zone d'investigation à l'orifice d'entrée ou aux orifices d'entrée par capillarité. Un tel corps fibreux peut être un matériau tissé ou non tissé.

Selon un mode de réalisation la pluralité de canaux microfluidiques comporte un canal microfluidique supplémentaire comportant un capteur électrochimique, le capteur électrochimique comportant au moins trois électrodes disposées de manière successive dans une direction longitudinale du canal microfluidique supplémentaire, les au moins trois électrodes comportant une électrode de référence, une contre-électrode et au moins une électrode de travail, le capteur électrochimique supplémentaire étant configuré pour polariser les électrodes à un potentiel électrique d'oxydation d'un composé chimique choisi parmi ion nitrite, peroxyde d'hydrogène et peroxynitrite et étant configuré pour produire au moins un signal représentatif d'une concentration dudit composé chimique dissout dans le flux de sueur.

Selon un mode de réalisation, le capteur électrochimique est configuré pour polariser au moins une dite électrode de travail pendant une durée déterminée avec une récurrence périodique.

Grâce à ces caractéristiques, il est possible d'obtenir une mesure de la concentration du monoxyde d'azote dissout dans le flux de sueur tout en diminuant le phénomène de passivation des électrodes.

Selon un mode de réalisation, les électrodes sont polarisées durant une durée comprise entre 1 seconde et 500 secondes avec une récurrence de périodicité comprise entre 1 minute et 60 minutes.

Grâce à ces caractéristiques, il est possible de mesurer la concentration d'une pluralité de composés chimiques.

Selon un mode de réalisation, une couche de poly-eugénol (4-allyl-2méthoxyphénol), d'un autre polyphénol ou d'un polymère similaire est déposée sur au moins une électrode de travail du capteur électrochimique. De préférence, le dépôt de la couche est effectué par électrochimie.

D'autres modes de réalisation des capteurs électrochimiques vont être décrits ci-dessous.

Selon un mode de réalisation permettant une détection séquentielle, le ou chaque ou au moins un dit capteur électrochimique est configuré pour détecter séquentiellement plusieurs espèces chimiques au cours d'une pluralité d'étapes de mesure, le capteur électrochimique étant configuré pour polariser les électrodes à un potentiel électrique d'oxydation du peroxyde d'hydrogène $H_2O_2$ au cours d'une première étape et polariser les électrodes à un potentiel électrique d'oxydation du monoxyde d'azote NO au cours d'une deuxième étape, et le capteur électrochimique est configuré pour produire un signal représentatif d'une concentration de monoxyde d'azote NO sur la base d'un premier signal de mesure ampérométrique obtenu lors de la première étape et d'un deuxième signal de mesure ampérométrique obtenu lors de la deuxième étape.

Avantageusement dans ce cas, le ou chaque ou au moins un dit capteur électrochimique est configuré pour polariser les électrodes à un potentiel électrique d'oxydation de l'ion nitrite $NO_2-$ au cours d'une troisième étape, et le capteur électrochimique est configuré pour produire un signal représentatif d'une concentration de l'ion nitrite $NO_2-$ sur la base desdits premier et deuxième signaux de mesure ampérométriques et d'un troisième signal de mesure ampérométrique obtenu lors de la troisième étape.

Selon un mode de réalisation, l'appareil de détection est réalisé de manière à pouvoir détecter séquentiellement trois des espèces chimiques précitées, avec un seul capteur électrochimique au cours d'une pluralité d'étapes de séquences temporelles de mesures. Selon ce mode de réalisation, lors d'une séquence donnée, le capteur électrochimique est configuré pour polariser une électrode en platine platiné (noir de platine) séquentiellement au potentiel électrochimique d'oxydation du peroxyde d'hydrogène $H_2O_2$ au cours d'une première étape temporelle de quelques secondes (5 s par exemple), puis à celui de l'oxydation du monoxyde d'azote NO au cours d'une deuxième étape temporelle de même durée, et éventuellement à celui de l'ion nitrite $NO_2-$ lors d'une troisième étape temporelle de même durée. Le capteur électrochimique est configuré pour produire un signal représentatif d'une concentration de monoxyde d'azote NO sur la base d'un premier signal de mesure ampérométrique obtenu lors de la première étape et d'un deuxième signal de mesure ampérométrique obtenu lors de la deuxième étape. Cette séquence se répète autant que nécessaire pendant la durée totale du test d'effort. La résolution d'un système de trois équations (les courants mesurés séquentiellement sur l'électrode polarisée à chaque potentiel dans une séquence) à deux ou trois inconnues (les concentrations en $H_2O_2$, NO et $NO_2-$) fournit les valeurs de chacune des trois concentrations au moment où chaque séquence est réalisée sur la base des trois mesures.

Selon un autre mode de réalisation permettant une détection simultanée, le appareil de détection comporte :

un premier canal microfluidique couplé à l'orifice d'entrée pour conduire un premier flux de sueur provenant de la zone d'investigation et un premier capteur électrochimique comportant des électrodes disposées dans le premier canal microfluidique, le premier capteur électrochimique étant configuré pour polariser les électrodes à un potentiel électrique d'oxydation du peroxyde d'hydrogène $H_2O_2$, et
un deuxième canal microfluidique couplé à l'orifice d'entrée pour conduire un deuxième flux de sueur provenant de la zone d'investigation et un deuxième capteur électrochimique comportant des électrodes disposées dans le deuxième circuit fluidique, le deuxième capteur électrochimique étant configuré pour polariser les électrodes à un potentiel électrique d'oxydation du monoxyde d'azote NO,
et le capteur électrochimique est configuré pour produire un signal représentatif d'une concentration de monoxyde d'azote NO sur la base d'un premier signal de mesure ampérométrique produit par le premier capteur électrochimique et d'un deuxième signal de mesure ampérométrique produit par le deuxième capteur électrochimique.

Avantageusement dans ce cas, l'appareil de détection comporte en outre :
un troisième canal microfluidique couplé à l'orifice d'entrée pour conduire un troisième flux de sueur provenant de la zone d'investigation et un troisième capteur électrochimique comportant des électrodes disposées dans le troisième circuit fluidique, le troisième capteur électrochimique étant configuré pour polariser les électrodes à un potentiel électrique d'oxydation de l'ion nitrite $NO_2^-$, et le capteur électrochimique est configuré pour produire un signal représentatif d'une concentration de l'ion nitrite $NO_2^-$ sur la base desdits premier et deuxième signaux de mesure ampérométriques et d'un troisième signal de mesure ampérométrique produit par le troisième capteur électrochimique.

Selon un mode de réalisation, l'appareil de détection comporte en outre :
un autre canal microfluidique, par exemple un quatrième canal microfluidique, couplé à l'orifice d'entrée pour conduire un autre flux de sueur provenant de la zone d'investigation, par exemple quatrième flux de sueur, et un autre capteur électrochimique, par exemple quatrième capteur électrochimique, comportant des électrodes disposées dans le quatrième canal microfluidique.

Selon ce mode de réalisation, ledit autre ou quatrième capteur électrochimique est configuré pour polariser les électrodes à un potentiel électrique d'oxydation du monoxyde d'azote et le quatrième canal microfluidique comporte un dépôt d'une couche de poly-eugénol sur l'électrode de travail du quatrième capteur électrochimique afin d'éliminer notamment le peroxyde d'hydrogène.

Selon un mode de réalisation, l'appareil comporte en outre une couche de matière adhésive recouvrant une face inférieure de la couche inférieure de la structure multicouche sans recouvrir l'orifice d'entrée de manière à former une barrière étanche autour de la zone d'investigation par contact avec l'épiderme dudit sujet.

Grâce à ces caractéristiques, les gaz, les liquides et les micro-organismes tels que bactéries ou virus, situés à l'extérieur de la zone d'investigation ne peuvent pas entrer dans la zone d'investigation. L'étanchéité du contact entre l'enveloppe et l'épiderme assure que l'espèce chimique détectée provient du liquide biologique produit par la zone d'investigation, et non d'un flux venant de l'extérieur.

Selon un mode de réalisation, la pluralité de canaux microfluidiques comporte un canal microfluidique supplémentaire comportant un dispositif de détection par colorimétrie, le dispositif de détection par colorimétrie comprenant un corps poreux hydrophile imprégné d'un réactif chimique capable de réagir avec un des composés chimiques suivants : ion nitrite, peroxyde d'hydrogène, peroxynitrite, dioxyde de soufre, sulfure d'hydrogène, monoxyde d'azote, monoxyde de carbone et acide hypochloreux, afin de fournir un indicateur coloré indiquant une concentration ou une quantité du composé chimique dissout dans le flux de sueur.

Grâce à ces caractéristiques, un suivi de concentration au cours du temps peut être effectué facilitant la lecture de l'utilisation. En outre, cela permet de consolider les résultats obtenus via le capteur électrochimique.

Selon un mode de réalisation, le canal supplémentaire comporte un système de chrono-échantillonnage relié à l'orifice d'entrée, le système de chrono-échantillonnage incluant une pluralité de chambres configurées pour se remplir de manière séquentielle de la sueur, et dans lequel une pluralité de dispositifs de détection par colorimétrie sont disposés dans lesdites chambres, chaque dispositif de détection par colorimétrie comprenant un réactif chimique capable de réagir avec un composé chimique, de sorte que les dispositifs de détection par colorimétrie disposés dans lesdites chambres fournissent un indicateur coloré indiquant une quantité cumulée dudit composé chimique dans le flux de sueur.

Un système de chrono-échantillonnage approprié est décrit notamment dans le document « Choi et al., Thin, Soft, Skin-Mounted Microfluidic Networks with Capillary Bursting Valves for Chrono-Sampling of Sweat Adv. Healthcare Mater. 2017 ».

Les structures de l'appareil de détection décrit précédemment peuvent être réalisées par différentes méthodes, par exemple sous la forme de structures multicouches. Elles peuvent aussi être obtenues par fabrication additive, impression 3D, laminage, ajout de matière en couches successives.

Selon un mode de réalisation, l'appareil de détection comporte en outre un capteur optique configuré pour produire un signal de mesure représentatif de l'intensité d'une couleur du réactif chimique dans le spectre visible ou ultraviolet.

Selon un mode de réalisation, l'appareil de détection est configuré pour effectuer et transmettre périodiquement des mesures, par exemple à une fréquence paramétrable ou à une fréquence dépendant d'un état d'activité détecté par l'appareil. Par exemple, l'appareil peut comporter un module gyroscopique et/ou d'un accéléromètre pour détecter l'état d'activité du sujet. Ainsi, il est possible de détecter l'état d'activité du sujet pendant les analyses de la sueur, afin de faciliter une analyse des corrélations entre l'état d'activité du sujet et la production des espèces chimiques analysées.

Selon un mode de réalisation, l'appareil comprend un module de géolocalisation.

Selon un mode de réalisation, l'appareil de détection comporte en outre un dispositif de communication configuré pour transmettre un ou plusieurs signaux de mesure produits par l'appareil de détection à destination d'un appareil de stockage ou de post-traitement.

Selon un deuxième objet, l'invention concerne Dispositif portatif comprenant un appareil de détection décrit précédemment, le dispositif portatif étant réalisé sous la forme de : une montre, un téléphone, un tissu, un bandeau, un vêtement, ou un sous vêtement.

Selon un mode de réalisation, les mesures produits par l'appareil de détection sont reçues, lues et analysées via une montre connectée et/ou un smartphone. La réception des mesures peut être effectuée par liaison filaire, infrarouge, Bluetooth, wifi, onde de type 3G, 4G ou 5G.

Selon un troisième objet, l'invention concerne un procédé pour déterminer la production d'au moins le monoxyde d'azote dissout dans la sueur par un sujet humain ou animal, le procédé comportant :

choisir une zone d'investigation d'un épiderme dudit sujet,
appliquer un appareil de détection précité pendant une durée nécessaire pour produire le signal représentatif d'une concentration du monoxyde d'azote NO dissout dans le flux de sueur et le signal représentatif d'une vitesse d'écoulement du flux de sueur, et
déterminer une mesure de la production du monoxyde d'azote NO par le sujet à partir du signal représentatif de la concentration du monoxyde d'azote NO dissout dans le flux de sueur.

Selon un mode de réalisation, le procédé comporte : désinfecter préalablement la zone d'investigation.

Grâce à cette caractéristiques, la mesure du monoxyde d'azote dans la sueur est davantage précise car elle ne comprend pas la fabrication de monoxyde d'azote par des bactéries et/ou virus présents sur la peau du sujet. C'est-à-dire que cela assure que le composé chimique détecté provient de la sueur produite par la zone d'investigation, et non d'un flux venant de l'extérieur.

Les mesures de la production d'une ou plusieurs des espèces chimiques précitées par le sujet peuvent être exploitées dans diverses applications, par exemple pour évaluer une souffrance des tissus vasculaires du sujet à partir de ces mesures ou pour évaluer une capacité cardiovasculaire du sujet à partir de ces mesures.

D'autres applications possibles sont le diagnostic, la prise en charge thérapeutique et le suivi de pathologies telles que maladie cardio-vasculaire, maladie neurodégé-nérative, hypertension artérielle pulmonaire, cancer, hyper-cholestérolémie, diabète, dysfonctionnement du système endothélial, artériosclérose, pathologie thrombotique ou ischémique, dysfonctionnement de l'inhibition d'accumulation plaquettaire ou de l'adhérence leucocytaire ou de la prolifération de cellules des fibres musculaires lisses, inflammation bronchique, asthme, maladie d'Alzheimer.

D'autres applications possibles sont la surveillance de la croissance et/ou de la souffrance musculaire d'une personne, par exemple une personne soumise à un entrainement physique, la prévention des blessures dues au surentrainement et/ou l'augmentation des performances musculaires du sujet.

**Brève description des figures**

Pour mieux faire comprendre l'objet de l'invention, on va en décrire ci-après, à titre d'exemple purement démonstratif et non limitatif, des modes de réalisation représentés sur les dessins annexés. Sur ces dessins :

[Fig.1] la [Fig.1] représente une vue schématique d'un sujet vu de dos sur lequel a été mis en place un appareil de détection selon un mode de réalisation,
[Fig.2] la [Fig.2] est une vue en perspective représentant partiellement une structure multicouche pour l'un appareil de détection selon un mode de réalisation,
[Fig.3] la [Fig.3] représente une vue en coupe selon la ligne II-II de la figure de la structure multicouche,
[Fig.4] la [Fig.4] est une vue éclatée de la structure multicouche selon un mode de réalisation,
[Fig.5] la [Fig.5] est une vue éclatée de la structure multicouche selon un autre mode de réalisation,
[Fig.6] la [Fig.6] est une vue agrandie en perspective d'un capteur électrochimique de la structure multicouche selon un mode de réalisation,

[Fig.7] la [Fig.7] est une vue éclatée de la structure multicouche selon un autre mode de réalisation,

[Fig.8] la [Fig.8] est une représentation schématique fonctionnelle d'un circuit microfluidique pouvant être utilisé dans un appareil de détection,

[Fig.9] la [Fig.9] est une autre représentation schématique fonctionnelle d'un autre circuit microfluidique pouvant être utilisé dans un appareil de détection,

[Fig.10] la [Fig.10] est une autre représentation schématique fonctionnelle d'un autre circuit microfluidique pouvant être utilisé dans un appareil de détection,

[Fig.11] la [Fig.11] est une représentation schématique en perspective d'un capteur électrochimique pouvant être utilisé dans le circuit microfluidique des figures 2 à 10,

[Fig.12] la [Fig.12] est un chronogramme illustrant un procédé de détection pouvant être effectué avec le capteur électrochimique de la [Fig.11],

[Fig.13] la [Fig.13] (A) représente un schéma d'un capteur électrochimique pouvant être utilisé dans le circuit microfluidique des figures 2 à 10 de l'appareil de détection selon un mode de réalisation, la [Fig.13] (B) représente un procédé de détection d'un débit de sueur pouvant être effectué avec le capteur électrochimique de la [Fig.13] (A),

[Fig.14] la [Fig.14] représente un schéma d'un capteur électrochimique de l'appareil de détection selon un mode de réalisation avec la fonction de déplétion,

[Fig.15] la [Fig.15] représente un schéma explicatif de la fonction de déplétion selon un mode de réalisation,

[Fig.16] la [Fig.16] est un chronogramme illustrant un procédé de détection pouvant être effectué avec le capteur électrochimique de la [Fig.14],

[Fig.17] la [Fig.17] illustre schématiquement un capteur électrochimique selon un mode de réalisation à cinq électrodes,

[Fig.18] la [Fig.18] illustre schématiquement un capteur électrochimique selon un mode de réalisation à six électrodes,

[Fig.19] la [Fig.19] illustre schématiquement un mode de réalisation de la structure multicouche comprenant en outre un dispositif de détection par colorimétrie,

[Fig.20] la [Fig.20] est une vue éclatée de la structure multicouche selon un autre mode de réalisation avec une pluralité de canaux,

[Fig.21] la [Fig.21] est une représentation schématique fonctionnelle d'un appareil de détection pouvant être utilisé dans l'appareil de la [Fig.1],

[Fig.22] la [Fig.22] est un diagramme d'étapes illustrant un procédé pouvant être mis en œuvre avec l'appareil de la [Fig.1],

[Fig.23] la [Fig.23] est un graphique illustrant un résultat des mesures pouvant être obtenues avec l'appareil de la [Fig.1].

## Description des modes de réalisation

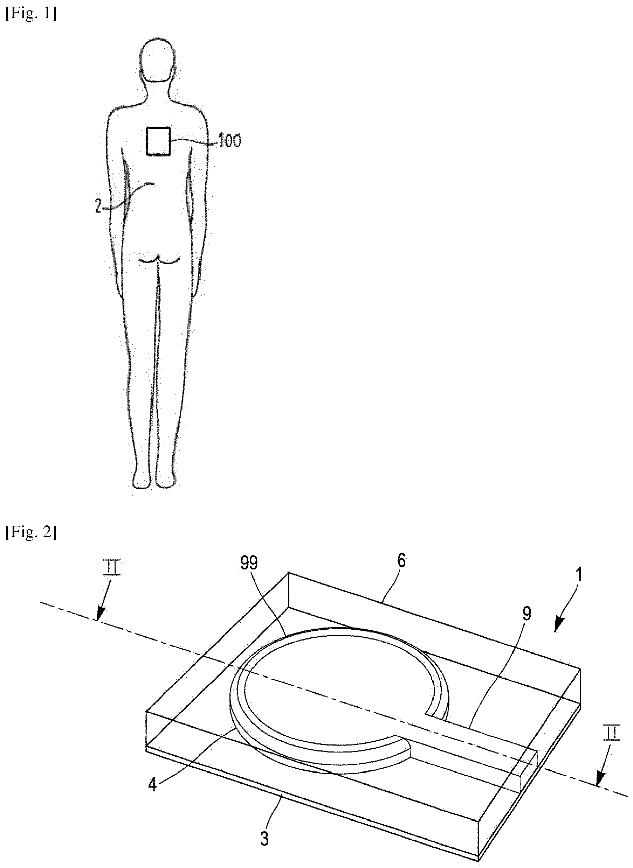

La [Fig.1] représente un appareil de détection 100 disposé sur la peau d'un sujet humain 2, par exemple dans le dos du sujet, et destiné à réaliser des mesures quantitatives d'espèces chimiques dissoutes dans la sueur, dont le monoxyde d'azote dissous dans la sueur, et éventuellement l'ion nitrite ou le peroxyde d'hydrogène. L'appareil de détection 100 peut être disposé sur une autre partie du corps, par exemple la nuque, l'épaule, le bras ou la jambe.

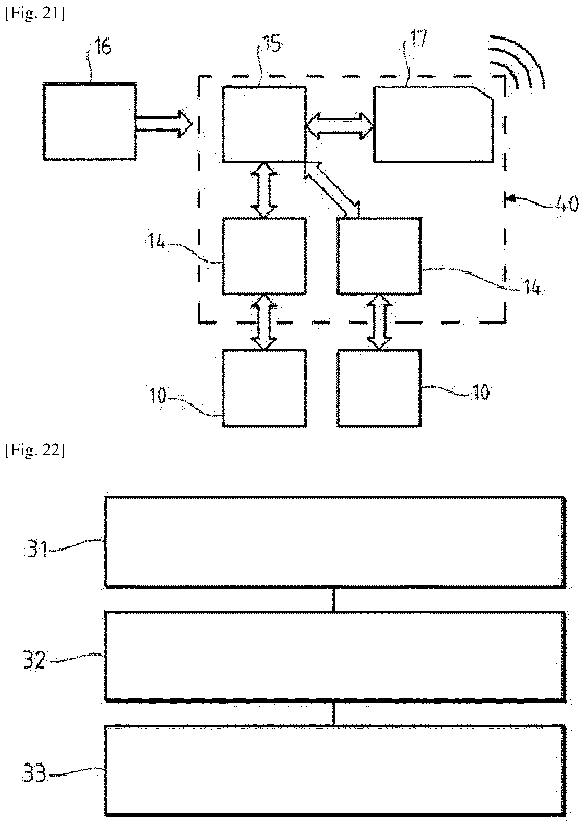

L'appareil de détection 100 comporte une partie microfluidique et d'autres parties fonctionnelles qui seront décrites plus bas, notamment un dispositif de contrôle 40 ( [Fig.21]).

En référence à la [Fig.2], la structure multicouche 1 se présente par exemple sous la forme d'un boitier peu encombrant qui comporte une couche inférieure 3 faite d'un matériau souple et biocompatible, de préférence autocollant, que l'on peut positionner directement sur la peau du sujet et une deuxième couche 6 qui est superposée à la couche inférieure 3. La structure multicouche 1 est faite de matériaux étanche à l'eau, par exemple fabriquée en polymère.

La deuxième couche 6 est creusée dans son épaisseur pour former un canal microfluidique 9 et une cupule de prélèvement 99 située au droit d'une ouverture 4 formée dans la couche inférieure 3.

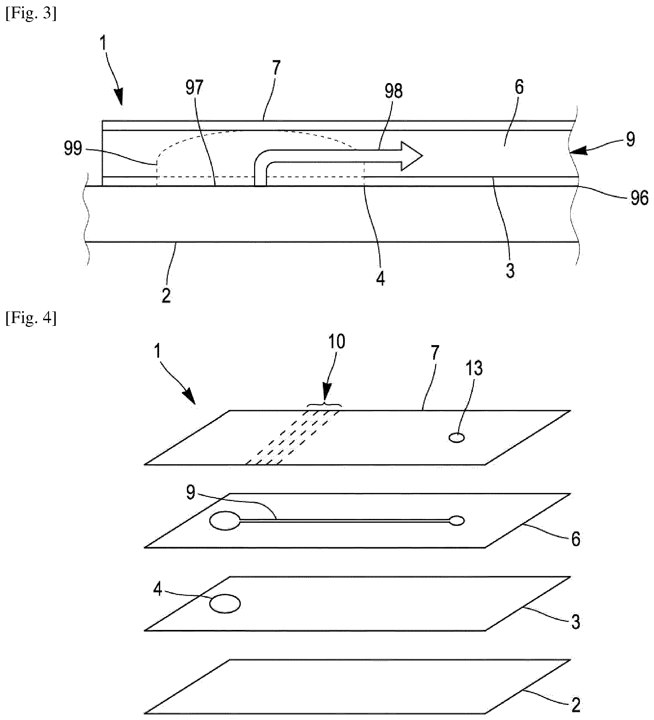

En référence à la [Fig.3], la couche inférieure 3 est collée sur la peau 2 par une couche adhésive 96. Une partie centrale de la couche inférieure 3 et de la couche adhésive 96 comporte l'ouverture circulaire 4 délimitant une zone d'investigation 97 sur la peau 2 du sujet, faisant par exemple quelques mm à quelques cm de diamètre. L'ouverture circulaire 4 peut prendre une autre forme, par exemple une ellipse, un triangle, un rectangle, un carré, un polygone ou autre. L'ouverture circulaire 4 est un orifice d'entrée permettant de guider un flux de sueur 98, notamment amener la sueur dans le canal microfluidique 9. Le flux de sueur 98 passe depuis la peau 2 du sujet jusque dans le canal microfluidique 9 en passant à travers l'ouverture circulaire 4.

Dans le mode de réalisation de la [Fig.3], une couche supérieure 7 recouvre la deuxième couche 6 pour former le circuit microfluidique par le haut. Le circuit microfluidique peut donc être formé à travers toute l'épaisseur de la deuxième couche 6, ce qui facilite sa fabrication, par exemple par découpe ou gravure.

Un élément collecteur hydrophile (non représenté), par exemple un corps fibreux, tel que du coton ou un matériau non tissé peut être disposé dans l'ouverture circulaire 4 et la cupule 99. L'élément collecteur remplit la fonction d'amener la sueur produite dans la zone d'investigation jusqu'au circuit microfluidique.

En référence à la [Fig.4], la structure multicouche comporte :

une couche inférieure 3 comprenant un orifice d'entrée 4 laissant passer la sueur,
une couche supérieure 7 comprenant un orifice de sortie 13,
une couche intermédiaire 6 située entre la couche inférieure 3 et la couche supérieure 7, le circuit microfluidique étant formé dans l'épaisseur de la au moins une couche intermédiaire 6 et s'étendant parallèlement à la couche inférieure 3.
Le circuit microfluidique est constitué d'un canal microfluidique 9 qui est en communication avec l'orifice d'entrée 4 en une première extrémité et en communication avec l'orifice de sortie 13 en deuxième extrémité. Ainsi, le flux de sueur provenant de la peau 2 du sujet est guidé dans le canal microfluidique 9 qui guide la sueur depuis l'orifice d'entrée 4 jusqu'à l'orifice de sortie 13 par capillarité.
Un capteur électrochimique 10 comporte quatre électrodes disposées sur la face interne de la couche supérieure 7 fermant le canal microfluidique par le haut. Ainsi, les électrodes se situent dans l'espace interne du canal microfluidique.

A titre d'exemple dimensionnel, l'orifice d'entrée présente un diamètre compris entre 1 mm et 15 mm, le canal microfluidique présente une longueur comprise entre 0,5 cm et 5 cm et une largeur comprise entre 25 $\mu$m et 500 $\mu$m, la couche intermédiaire présente une épaisseur comprise entre 10 $\mu$m et 200 $\mu$m, les couches de la structure multicouche présentent une largeur comprise entre 1 cm et 5 cm et une longueur comprise entre 2 cm et 15 cm.

Par exemple, l'orifice d'entrée 4 présente un diamètre de 5 mm, le canal microfluidique 9 présente une longueur de 1,8 cm et d'une largeur de 100 $\mu$m, la couche intermédiaire présente une épaisseur inférieure à 70 $\mu$m par exemple 20 $\mu$m, les couches de la structure multicouche présentent une largeur de 3 cm et une longueur de 9 cm.

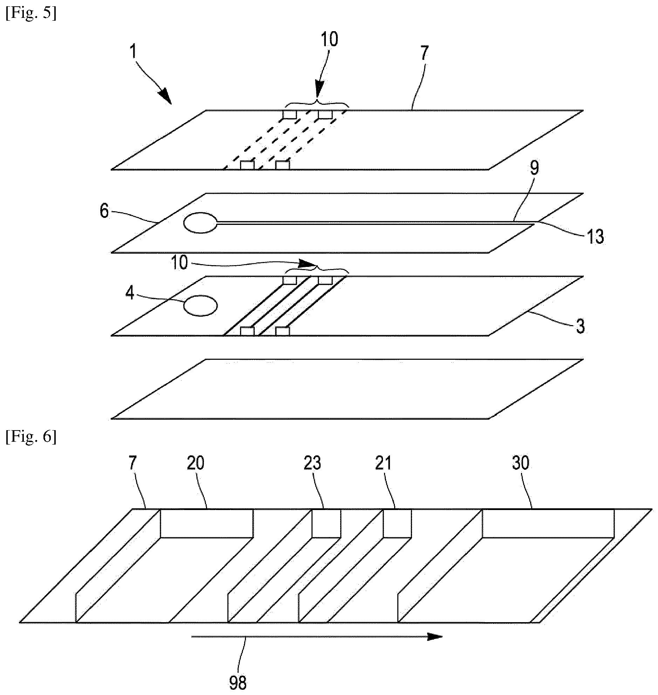

En référence à la [Fig.5], la structure multicouche est similaire à la [Fig.4]. Cependant, dans ce mode de réalisation, l'orifice de sortie 13 est situé dans la couche intermédiaire 6, en une extrémité de la couche intermédiaire. L'appareil de détection 100 comporte un capteur électrochimique 10 comportant respectivement quatre électrodes, chaque électrode comporte respectivement deux parties disposées en vis à vis, une première partie disposée sur une face interne de la couche supérieure 7 fermant le canal microfluidique 9 par le haut et une deuxième partie disposée sur une face supérieure de la couche inférieure 3 fermant le canal microfluidique par le bas. Chaque partie d'électrode comprend un connecteur illustré par un rectangle noir permettant de relier électriquement les électrodes.

Selon un mode de réalisation non illustré, l'appareil de détection peut comporter un unique capteur électrochimique 10 comportant quatre électrodes disposées sur une face supérieure de la couche inférieure 3 fermant le canal microfluidique 9 par le bas.

La [Fig.6] illustre quatre électrodes arrangées dans un canal microfluidique, pouvant être le canal microfluidique 9 présenté sur les figures 4 et 5 par exemple. Les quatre électrodes sont des dépôts métalliques disposées sur la face interne de la couche supérieure 7 fermant le canal microfluidique 9 par le haut. Les quatre électrodes disposées de manière successive dans une direction longitudinale du canal microfluidique et comportent : une électrode de travail 20 réalisée ici sous la forme d'une électrode de déplétion dont le fonctionnement sera détaillé plus bas, une deuxième électrode de travail 23, une électrode de référence 21 et une contre-électrode 30. La hauteur des électrodes est comprise entre 1 et 50 nano mètre (nm), l'espace entre les électrodes est compris entre 10 et 10000 micromètre ($\mu$m) et la largeur des électrodes est comprise entre 1 et 1000 $\mu$m. Les électrodes peuvent être fabriquées par exemple en platine (Pt), en Or (Au), en argent (Ag), en chlorure d'argent (AgCl).

Au moins une des électrodes peut également être recouverte en totalité ou en partie de poly-eugénol, de noir de platine ou de polyphenol. Les électrodes sont configurées pour effectuer une ou plusieurs de ces actions : dépléter, mesurer la concentration du monoxyde d'azote, mesurer la concentration d'au moins un autre composant chimique et mesurer le débit du flux de sueur s'écoulant dans le canal microfluidique 9.

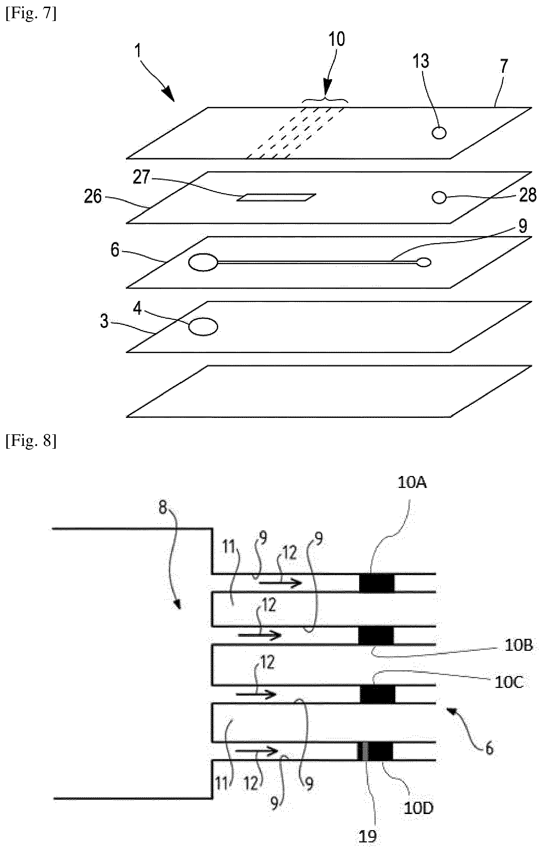

La [Fig.7] illustre la structure multicouche 1 similaire à la [Fig.4], dans laquelle une deuxième couche intermédiaire d'étanchéité 26 est située entre la première couche intermédiaire 6 et la couche supérieure 7 et la deuxième couche intermédiaire d'étanchéité 26 comporte une ouverture 27 au niveau des électrodes permettant de mettre en contact les électrodes avec le flux de sueur circulant dans le canal microfluidique 9. La deuxième couche intermédiaire d'étanchéité 26 comporte en outre une ouverture intermédiaire 28 communiquant avec l'orifice de sortie 13 de la couche supérieure 7 afin de permettre l'évacuation de la sueur. Par exemple, l'ouverture 27 est en forme de rectangle et présente une longueur de 5 mm et une largeur de 200 $\mu$m.

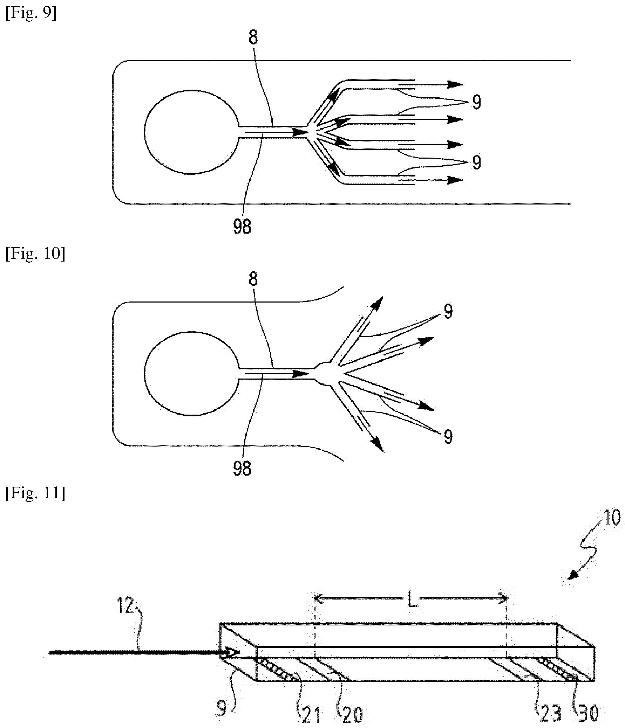

En référence à la [Fig.8], la couche intermédiaire 6 comporte un circuit microfluidique 8 alimenté par un flux de sueur. La sueur est reçue depuis l'orifice d'entré de la couche inférieure vers le circuit microfluidique 8. Le circuit microfluidique 8 peut comporter un ou plusieurs canaux microfluidiques 9, à savoir quatre canaux microfluidiques 9 parallèles dans l'exemple représenté. Cependant, le circuit microfluidique 8 peut prendre différentes formes, par exemple est représenté à la [Fig.9] un circuit microfluidique 8 comportant des canaux microfluidiques 9 parallèles et à la [Fig.10] quatre canaux microfluidiques 9 distribués radialement. Les canaux microfluidiques 9 sont par exemple formés dans l'épaisseur de la couche intermédiaire 6 et séparés par des cloisons 11. Chaque canal microfluidique 9 est respectivement séparé des autres canaux microfluidique au sein desquels la sueur peut circuler de manière indépendante. Le nombre de canaux microfluidiques 9 peut être plus élevé ou plus faible que sur ces figures.

Chaque circuit fluidique 9 est muni d'un capteur 10A, 10B, 10C ou 10D. Les flèches 12 illustrent le sens d'écoulement de la sueur dans les canaux microfluidiques 9. De préférence, les canaux microfluidiques 9 aboutissent via l'orifice de sortie 13 dans un réservoir de drainage qui retient les fluides analysés, de manière à éviter de remettre les produits de réaction de l'électrolyse en contact avec la peau du sujet.

Les capteurs 10A, 10B, 10C et 10D agencés dans les circuits microfluidiques 9 pour analyser la sueur sont de préférence des capteurs électrochimiques. Le principe de fonctionnement d'un capteur électrochimique est d'électrolyser en partie ou en totalité la solution présente dans le canal fluidique 9 entre une électrode de travail et une contre-électrode. Un tel capteur électrochimique peut être réalisé de différentes manières, notamment de manière miniaturisée avec des dimensions de l'ordre du millimètre.

On va maintenant décrire plusieurs exemples de réalisation des capteurs électrochimiques en référence à la [Fig.8].

**Exemples**

Exemple 1

Le capteur 10A est destiné à détecter le peroxyde d'hydrogène. Il fonctionne donc avec une différence de potentiel égale au potentiel d'oxydation du peroxyde d'hydrogène $E_{H2O2}$. Le capteur 10B est destiné à détecter le monoxyde d'azote. Il fonctionne donc avec une différence de potentiel égale au potentiel d'oxydation du monoxyde d'azote $E_{NO}$. Le capteur 10C est destiné à détecter l'ion nitrite. Il fonctionne donc avec une différence de potentiel égale au potentiel d'oxydation de l'ion nitrite $E_{NO2^-}$.

Les capteurs 10A, 10B, 10C effectuent des mesures synchrones des intensités instantanées, notées $i_{oxdn}$, des courants Faradiques liés à l'oxydation électrochimique des espèces chimiques précitées. Les capteurs 10a, 10B, 10C permettent ainsi la détection et quantification de la concentration instantanée des espèces chimiques précitées.

Chacune des trois espèces chimiques précitées est détectable par des mesures ampérométriques à l'aide de microélectrodes. Celles-ci sont par exemple constituées de bandes de platine recouvertes d'une fine couche, par exemple micrométrique, de noir de platine déposé par réduction électrochimique en milieu aqueux de l'anion d'un sel de platine, $Pt(Cl)_6^{4-}$.

Les trois espèces chimiques ($NO$, $NO_2^-$ et $H_2O_2$) sont distinguables grâce au fait que leurs potentiels d'oxydation sur ces électrodes sont bien séparés, intervenant dans l'ordre suivant : $E_{H2O2} < E_{NO} < E_{NO2^-}$. Cependant les courants Faradiques sont additifs. Le courant mesuré au potentiel d'oxydation de chaque espèce chimique additionne donc des courants élémentaires liés à l'oxydation de cette espèce chimique et à l'oxydation de toutes les espèces chimiques dont les potentiels d'oxydation sont inférieurs.

Ainsi, seule l'espèce $H_2O_2$ est oxydable au potentiel d'oxydation $EH_2O_2$. Les espèces $H_2O_2$ et NO sont oxydables au potentiel d'oxydation $E_{NO}$. Les trois espèces sont oxydables au potentiel d'oxydation $E_{NO2^-}$. Les courants mesurés par les capteurs 10A à 10C, notés respectivement $i_{oxdn}(EH_2O_2)$, $i_{oxdn}(E_{NO})$ et $i_{oxdn}(E_{NO2^-})$ obéissent donc aux équations suivantes :

$$i_{oxdn}(E_{H2O2}) = a1\ i_{H2O2}$$

$$i_{oxdn}(E_{NO}) = a2\ i_{H2O2} + a3\ i_{NO}$$

$$i_{oxdn}(E_{NO2}^-) = a4\ i_{H2O2} + a5\ i_{NO} + a6\ i_{NO2^-}$$

où les coefficients a1 à a6 représentent des constantes de calibration des capteurs, pouvant être mesurées expérimentalement.

On obtient ainsi par des soustractions facilement implémentées sur un circuit électronique :

$$i_{\text{H2O2}} = (1/a1)\ i_{\text{oxdn}}\ (E_{\text{H2O2}})$$

$$i_{\text{NO}} = (1/a3)\ i_{\text{oxdn}}\ (E_{\text{NO}})\ \text{-}\ (a2/a1).(1/a3)\ i_{\text{oxdn}}\ (E_{\text{H2O2}})$$

$$i_{\text{NO2-}} = (1/a6)\ i_{\text{oxdn}}\ (E_{\text{NO2-}})\ \text{-}\ (a4/a6)\ i_{\text{H2O2}} - (a5/a6)\ i_{\text{NO}}$$

A tout instant t, l'intensité instantanée du courant Faradique d'oxydation, $i_s(t)$, de chaque espèce chimique S est proportionnelle à sa concentration, $C_s(t)$, dans le volume de fluide situé au-dessus des électrodes qui la détectent. Le facteur de proportionnalité dépend d'un facteur de forme, noté $\gamma$, qui est une fonction de la géométrie du capteur, et du nombre de Faraday, noté $n_s$, consommé par mole de l'espèce chimique, à savoir :

$n_{\text{H2O2}} = n_{\text{NO2-}} = 2$ et $n_{\text{NO}} = 1$

On rappelle que F désigne le Faraday, c'est-à-dire 96 500 Coulombs, la valeur de la charge d'une mole d'électrons.

Le facteur de forme y est un facteur constant imposé par la géométrie du capteur électrochimique, évaluable théoriquement et mesurable expérimentalement par calibration. Par mesure de simplicité, on considère ci-dessous que les trois capteurs 10A à 10C ont des géométries identiques et de sorte que le facteur de forme y est commun à tous les capteurs.

Il en résulte que les concentrations des espèces chimiques peuvent être obtenues à partir des courants mesurés par les capteurs 10A à 10C, à l'aide des expressions suivantes, où la variable temporelle t a été explicitée :

$$C_{\text{H2O2}}(t) = i_{\text{oxdn}}(E_{\text{H2O2}},\ t)/(2F\gamma)$$

$$C_{\text{NO}}(t) = [i_{\text{oxdn}}(E_{\text{NO}},\ t)\ \text{-}\ i_{\text{oxdn}}\ (E_{\text{H2O2}},\ t)]/(F\gamma)$$

$$C_{\text{NO2-}}(t) = [i_{\text{oxdn}}(E_{\text{NO2-}},\ t)\ \text{-}\ i_{\text{oxdn}}(E_{\text{NO}},\ t)]/(2F\gamma)$$

Dans l'exemple 1, les trois capteurs 10A à 10C peuvent donc fonctionner en parallèle, chacun avec un potentiel d'oxydation constant, à savoir $E_{\text{H2O2}}$, $E_{\text{NO}}$, et $E_{\text{NO2-}}$ respectivement.

Selon une variante de réalisation, il est détecté uniquement le NO et le $NO_2^-$. Ce mode de réalisation est particulièrement avantageux lorsque la mesure de $H_2O_2$ n'est pas significative et n'influence pas les résultats de l'objectif visé. La concentration $C_{\text{H2O2}}(t)$ présentée ci-dessus est alors considérée comme uniformément nulle, soit $C_{\text{H2O2}}(t) = 0$. Le système d'équations est donc simplifié.

Exemple 2

Dans l'exemple 2, un seul canal microfluidique 9 et un seul capteur 10A sont utilisés, les autres pouvant être supprimés.

Dans ce cas, le capteur 10A fonctionne séquentiellement pour détecter les espèces chimiques précitées au cours de trois étapes successives. Le potentiel d'oxydation est donc commuté entre trois paliers de potentiel respectivement égaux aux trois potentiels d'oxydation précités, par exemple périodiquement selon la séquence $E_{\text{H2O2}} \rightarrow E_{\text{NO}} \rightarrow E_{\text{NO2-}} \rightarrow E_{\text{H2O2}} \rightarrow E_{\text{NO}} \rightarrow E_{\text{NO2-}} \rightarrow$ etc.

Dans ce cas, chaque potentiel d'oxydation est maintenu pendant une durée très grande par rapport à la constante de temps de l'électrode de travail, cette constante de temps étant par exemple quelques millisecondes pour des microélectrodes implémentées dans des canaux microfluidiques, et des mesures du courant sont faites à la fin de chaque palier de potentiel.

Pour le reste, le traitement des signaux de mesure peut être réalisé selon les mêmes équations qu'à l'exemple 1.

Exemple 3

Le monoxyde d'azote étant une petite molécule à la fois hydrophile et lipophile, elle peut aisément traverser de fines couches de polymère organique, au contraire des deux autres espèces $H_2O_2$ et $NO_2^-$. Elle peut donc être détectée isolément à l'aide d'un capteur électrochimique protégé par une telle couche, par exemple avec une électrode de travail en platine platiné revêtue une fine couche de poly-eugénol (4-allyl-2-méthoxyphénol) déposée par électro-polymérisation.

Dans l'exemple 3, l'électrode de travail du capteur 10D est ainsi revêtue par la couche représentée schématiquement au chiffre 19. La concentration instantanée de monoxyde d'azote peut ainsi être mesurée indépendamment de celle des espèces chimiques $H_2O_2$ et $NO_2$-, selon l'expression :

$$C_{\mathrm{NO}}(t) = [i_{\mathrm{oxdn}}(E_{\mathrm{NO}},t)]_{\mathrm{eugénol}}/(F\gamma) \ .$$

Où $i_{\mathrm{oxdn}}(E_{\mathrm{NO}}, t)]_{\mathrm{eugénol}}$ désigne le courants mesuré par le capteur 10D.

Les autres capteurs 10A à 10C et les autres canaux microfluidiques 9 peuvent être supprimés. Cette méthode peut donc être avantageusement utilisée avec un seul capteur lorsque seule la concentration de NO est recherchée.

Exemple 4

On cumule dans ce cas le capteur 10D de l'exemple 3 avec les capteurs 10A à 10C de l'exemple 1 ou avec le capteur 10A de l'exemple 2. Cette configuration permet d'obtenir deux mesures indépendantes de la concentration du monoxyde d'azote dissous et donc de contrôler la cohérence des mesures en vérifiant en particulier l'absence de dérive des capteurs, par exemple liée à une désactivation partielle de la surface de l'une des électrodes.

Dans ce cas, le dispositif électronique de contrôle électrochimique 40 ([Fig.21]) est de préférence configuré pour comparer les deux mesures de la concentration du monoxyde d'azote et émettre une alarme lorsque le résultat de la comparaison remplit un critère prédéfini, par exemple dépasse un seuil prédéfini.

Dans les exemples 1 à 4 ci-dessus, les courant Faradiques instantanés mesurés permettent de mesurer la concentration des espèces chimiques dans la solution analysée. Par conséquent, dans un système statique, l'intensité du courant suffit à rendre compte de la production de l'espèce détectée.

Cependant, lorsque l'appareil de détection 100 est appliqué à un système physiologique essentiellement dynamique, il est souhaitable de pouvoir aussi accéder quantitativement à la dynamique de production de chaque espèce chimique par le système cardiovasculaire, par exemple lors de tests d'effort ou lors de suivis médicaux. Dans des conditions dynamiques, pour accéder à la quantité instantanée d'une espèce chimique, notée $\Delta Q(t)$, produite pendant une petite durée de temps, notée $\Delta t(t)$, il est souhaitable de connaître simultanément la concentration moyenne, $C_s(t)$, de l'espèce chimique et le débit volumique du fluide analysé, à savoir :

$$d(t) = (\Delta V/\Delta t)$$

où $\Delta V$ désigne le volume balayé pendant l'intervalle de temps $\Delta t$. Ainsi, l'intensité du flux de production, notée $P_s(t)$, d'une espèce chimique S à un instant t est donnée par :

$$P_S(t) = [\Delta Q/\Delta t](t) = C_S(t).d(t)$$

où la concentration moyenne $C_s(t)$ est obtenue à partir des intensités moyennes des courants d'oxydation électrochimique mesurés entre les instants t et t+$\Delta t$.

Dans le cadre des applications dynamiques envisagées, il est donc souhaitable que l'appareil de détection 100 mesure à la fois et à chaque instant t requis par la précision souhaitée pour le suivi temporel de l'état physiologique du patient, par exemple une fois par minute, les intensités moyennes, $i_{\mathrm{av}}(t)$, du courant Faradique lié à l'oxydation électrochimique de la ou des espèces chimiques suivies et la valeur du débit volumique d(t) de sueur au temps t dans le circuit fluidique correspondant.

La [Fig.11] illustre un mode de réalisation d'un capteur électrochimique 10 pouvant répondre à ce double besoin de manière intégrée. Ce capteur électrochimique 10 comporte au moins une paire d'électrodes de travail 20, 23. Une telle microélectrode bande peut être réalisée en platine platiné (noir de platine), recouverte ou non d'une couche micrométrique d'eugénol électropolymérisée. Une telle microélectrode bande peut être implantée par micro-fabrication, par exemple CVD et/ou lithographie. Cette ou ces microélectrodes bandes permettent d'oxyder électrochimiquement les espèces chimiques choisies.

Le circuit microfluidique 9 de la [Fig.11] est en outre équipé d'une électrode de référence 21, réalisée par exemple sous la forme d'une microbande d'Ag/AgCl, et placée en amont de la paire d'électrodes de travail 20, 23. Le circuit fluidique 9 est enfin équipé d'une contre-électrode 30 en platine platiné placée en aval de la paire d'électrodes de travail 20, 23. Nonobstant la représentation schématique fonctionnelle de la [Fig.11], la surface de la contre-électrode 30 est en réalité deux à trois fois plus grande que celle des autres électrodes.

L'ensemble du canal microfluidique 9 avec les électrodes 20, 21, 23, 30 est baigné par une lame de sueur non représentée, et constitue ainsi une cellule électrochimique microfluidique à quatre électrodes. Chacune des électrodes 20, 21, 23, 30 est connectée avec un dispositif électronique de contrôle électrochimique 40 ([Fig.21]) grâce à des contacts électriques isolés de la sueur.

Ce mode de réalisation d'un capteur électrochimique 10 peut être employé dans un ou plusieurs des circuits microfluidiques 9 précités.

Pour mesurer le débit volumique d(t), le capteur électrochimique 10 doit comporter la paire d'électrodes de travail 20, 23. La solution décrite ici est simple et facilement industrialisable, car sans pièce mobile ni aucune hypothèse sur le régime hydrodynamique. Elle ne nécessite aucune intervention visant à moduler le débit du fluide, tout en étant adaptée à tout débit physiologique raisonnable.

Les deux électrodes de travail 20, 23, par exemple deux bandes de platine platiné pouvant servir de microélectrodes de travail, sont électriquement indépendantes et sont espacées d'une distance L le long de la trajectoire du fluide analysé dans le circuit microfluidique 9. Les deux électrodes de travail 20 et 23 sont par exemple implantées sur le fond d'un canal linéaire dont la section présente une aire constante A.

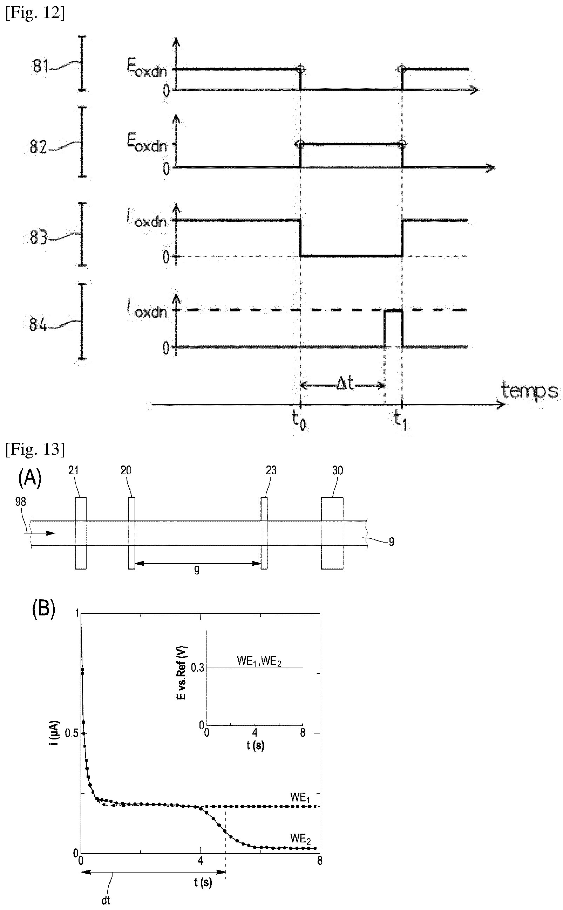

L'électrode de travail 23 placée en aval est utilisée selon le procédé illustré sur la [Fig.12], qui comporte deux étapes. Le graphique 81 représente le potentiel électrique appliqué à l'électrode de travail 20 en fonction du temps. Le graphique 82 représente le potentiel électrique appliqué à l'électrode de travail 23 en fonction du temps. Les potentiels indiqués comme '0' sur les graphiques 81 et 82 signifient en réalité une déconnexion de l'électrode correspondante (circuit ouvert). Le graphique 83 représente le courant faradique mesuré à l'électrode de travail 20 en fonction du temps. Le graphique 84 représente le courant faradique mesuré à l'électrode de travail 23 en fonction du temps.

Pendant une première étape effectuée sur une plage de temps antérieure à l'instant $t_0$, le potentiel $E_{oxdn}$ appliqué à l'électrode de travail 20 est adéquat pour permettre l'oxydation de la ou des espèces chimiques visées, tandis que l'électrode de travail aval 23 est déconnectée. L'électrode de travail 20 placée en amont permet alors d'enregistrer en continu le courant électrochimique instantané, $i_{oxdn}(t)$, indique ainsi, à la suite des éventuels calculs indiqués plus haut, la concentration $C(t)$ de la ou des espèces chimiques visées dans le fluide analysé.

Pendant une deuxième étape effectuée sur une plage de temps à partir de l'instant t0, l'électrode de travail 20 est déconnectée et le potentiel $E_{oxdn}$ est appliqué à l'électrode de travail 23 aval.

A l'instant $t_0$, le flux de sueur passant au-dessus de l'électrode de travail 23 a déjà été électrolysé (complètement ou en partie) lors de son passage au-dessus de l'électrode de travail 20 qui est située en amont, de sorte que la concentration de l'espèce chimique visée y est nulle ou tout au moins bien plus faible qu'avant son entrée dans le capteur électrochimique. L'intensité $i_{oxdn}$ du courant détecté par l'électrode de travail 23 (graphique 84) est donc nulle (ou tout au moins bien plus faible que celle du courant i $_{oxdn}$ détecté à l'électrode de travail 20 avant l'instant $t_0$.

A l'instant $t_0+\Delta t$ l'électrode de travail 23 commence à analyser une solution non électrolysée et l'intensité du courant $i_{oxdn}$ qu'elle détecte devient du même ordre que celle détectée par l'électrode de travail 20 avant l'instant $t_0$. La croissance du courant, schématisé par un échelon sur la [Fig.12], est détectée par un circuit électronique ad-hoc. La durée $\Delta t$, qui est le retard entre cette croissance et le moment t0 de la déconnection de l'électrode de travail 20, représente le temps nécessaire au flux de sueur pour transiter entre les deux électrodes de travail 20 et 23. La durée $\Delta t$ est représentée par une double flèche en bas de la [Fig.12]. Afin de simplifier la représentation, on a supposé sur la [Fig.12] que l'électrolyse de l'espèce chimique visée est complète lorsque l'électrode de travail 20 est connectée. Les mêmes principes de mesure sont applicables lorsque cette électrolyse est seulement partielle.

La vitesse de l'écoulement v(t) et le débit d(t) peuvent donc être estimé comme suit :

$$v(t) = L/\Delta t$$

$$d(t) = A.v(t)$$

Le potentiel $E_{oxdn}$ appliqué à l'électrode de travail 23 est adéquat pour permettre l'oxydation de la ou des espèces chimiques visées, tandis que l'électrode de travail 20 est déconnectée. La mesure de concentration peut donc éventuellement se poursuivre pendant une certaine durée avec l'électrode de travail 23. La deuxième étape se termine par la déconnexion de l'électrode de travail 23 à l'instant $t_1$. L'électrode de travail 20 peut être alors reconnectée et le procédé peut être réitéré autant que de nécessaire pour évaluer le débit d(t) à des instants successifs.

La distance L entre les deux électrodes de travail 20 et 23 est de préférence suffisamment petite, par exemple de l'ordre de 1 mm, pour que les changements de la réponse physiologique du patient soient négligeables pendant la durée $\Delta t$.

Une deuxième méthode de mesure du débit volumique de l'écoulement de la sueur est illustrée sur la [Fig.13]. Contrairement à l'explication ci-dessus, le principe de mesure consiste ici à détecter une chute du courant de mesure d'un composé chimique, par exemple du monoxyde d'azote. La [Fig.13] A illustre schématiquement un ensemble de quatre électrodes disposées dans un canal microfluidique 9 selon un mode de réalisation. Les électrodes sont disposées de manière successive dans une direction longitudinale du canal microfluidique 9. Les électrodes sont disposées de la manière suivante : une électrode de référence 21 disposée en amont et recevant en premier le flux de sueur 98, une première et une deuxième électrodes de travail 20, 23 espacées d'une distance g représentée par la double flèche et une contre-électrode 30. Le principe de mesure est illustré sur la [Fig.13] (B) et consiste à connecter et à polariser les deux électrodes de travail 20, 23 simultanément, puis à suivre en fonction du temps l'évolution du courant de la deuxième électrode de travail 23 située en aval dans le canal microfluidique 9. Dès le début de la polarisation (à t=0), les deux électrodes de travail 20, 23 oxydent les mêmes espèces. La première électrode de travail 20, en amont, crée une déplétion de ces espèces et induit après une durée dt une diminution du courant de la seconde électrode de travail 23 située en aval. dt est le temps nécessaire pour que la zone de déplétion comprenant l'espèce oxydée par la première électrode de travail 20 atteigne par convection la seconde électrode 23. On observe ainsi qu'après la polarisation simultanée de la première 20 et de la deuxième électrode de travail 23 à t=0, le courant de la deuxième électrode de travail 23, située en aval, diminue à la durée dt. A titre d'exemple, la durée dt de la [Fig.13] (B) est estimée à cinq secondes.

Ainsi, la vitesse linéaire v de l'écoulement est obtenue par la simple relation v = g/ dt.

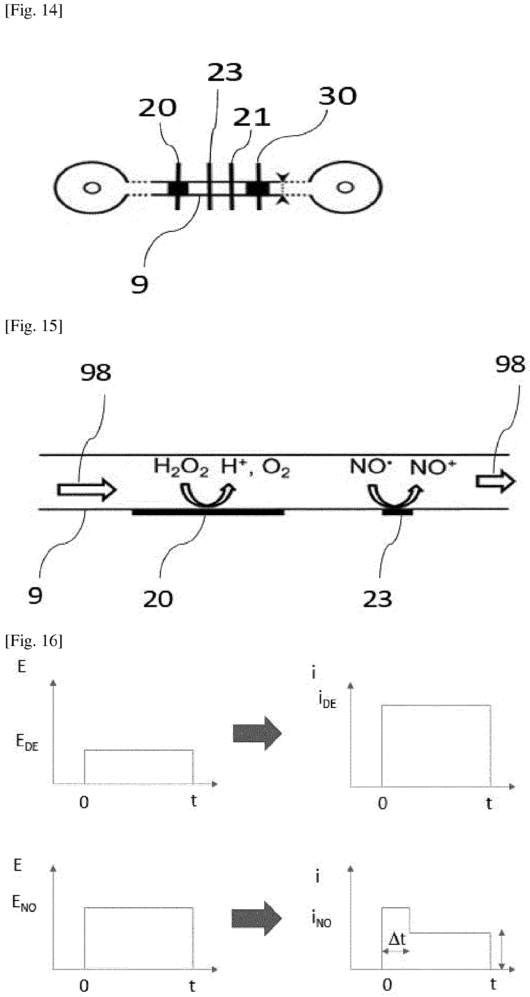

Ce principe peut être exploité en combinaison avec une fonction de déplétion décrite en référence aux figures 14 à 16 ci-après.

La [Fig.14] illustre le circuit microfluidique comportant un capteur électrochimique comprenant quatre électrodes disposées de manière successive dans une direction longitudinale du canal microfluidique 9. Une électrode de déplétion 20 est placée en amont de l'électrode de travail 23, pour la déplétion des espèces interférentes et dont la détection en aval n'est pas souhaitée. Par polarisation de l'électrode de déplétion 20, les espèces chimiques interférentes sont oxydées sélectivement. L'électrode de déplétion 20 est large afin d'optimiser la déplétion et d'éliminer la quasi-totalité de ou des espèces interférentes, par exemple éliminer le peroxyde d'hydrogène $H_2O_2$. Les espèces chimiques d'intérêts seront oxydées par l'électrode de travail 20 située en aval. Une telle configuration permet la déplétion d'une ou plusieurs espèces chimiques interférentes présentant un potentiel d'oxydation inférieur au potentiel d'oxydation du monoxyde d'azote et permet d'améliorer la mesure d'une concentration d'un composé chimique, par exemple le monoxyde d'azote. La contre électrode 30 et l'électrode de référence 20 sont nécessaires pour le contrôle des potentiels et la circulation des courants dans la zone de mesure du capteur électrochimique.

Dans le canal microfluidique 9 muni de cette électrode de déplétion 20, on peut obtenir directement une concentration du monoxyde d'azote sans avoir besoin de résoudre le système d'équations linéaires présenté plus haut.

Dans une variante de réalisation où l'on souhaite obtenir la mesure de la concentration du $NO_2^-$ dans le canal microfluidique 9, l'électrode de déplétion 20 peut être configurée pour éliminer le monoxyde d'azote.

La [Fig.15] présente un exemple de déplétion du peroxyde d'hydrogène avec le capteur de la [Fig.14]. L'électrode de référence et la contre-électrode ne sont pas représentées. L'électrode de déplétion 20 et l'électrode de travail 23 sont polarisées indépendamment l'une de l'autre dans des conditions de débit du flux de sueur constant. Le sens du débit de sueur est représenté via les flèches dans le canal microfluidique 9. L'électrode de déplétion 20 est très grande par rapport à l'électrode de travail 23, par exemple huit fois plus grande afin d'éliminer en amont la totalité de l'espèce interférente, ici le peroxyde d'hydrogène ($H_2O_2$) par oxydation. L'espèce chimique dont on souhaite mesurer la concentration, ici le monoxyde d'azote n'est pas oxydée par l'électrode de déplétion 20. Le monoxyde d'azote sera oxydé en aval par l'électrode de travail 23. Ainsi, l'électrode de déplétion 20 et l'électrode de travail 23 sont polarisées à des potentiels tels que $E_{DE} < E_{WE}$, afin d'obtenir la sélectivité de la détection du monoxyde d'azote.

Comme énoncé ci-dessus, la mesure du débit du flux de sueur peut être effectuée via l'électrode de travail 20 effectuant la déplétion et l'électrode de travail 23 oxydant le monoxyde d'azote par exemple. La [Fig.16] illustre une mesure du débit du flux de sueur 98 selon une méthode similaire à celle décrite sur la [Fig.13] A et B. Ainsi, l'électrode de déplétion 20 oxydant l'espèce chimique que l'on ne souhaite pas détecter, par exemple le peroxyde d'hydrogène et l'électrode de travail 23 oxydant le monoxyde d'azote sont connectées et polarisées simultanément. Ainsi, dans un premier temps l'électrode de travail 23 détectera le monoxyde d'azote NO ainsi que le peroxyde d'hydrogène car le volume de sueur situé entre l'électrode de déplétion 20 et l'électrode de travail 23 ne sera pas dépiété par l'électrode de déplétion 20. Dans un deuxième temps, l'électrode de déplétion 20 crée une déplétion du peroxyde d'hydrogène ce qui induit, de manière similaire à ce qui est décrit avec la [Fig.13], après une durée d\.une diminution du courant de l'électrode de travail 23. Dans ce mode de réalisation le courant de l'électrode de travail 23 diminue mais ne devient pas nul ou quasiment nul car l'électrode de travail 23, à la suite de la déplétion, détecte sélectivement le monoxyde d'azote.

Les procédés de mesure de débit décrit ci-dessus peuvent être employés simultanément dans tous les canaux microfluidiques parallèles. Toutefois, si ces canaux sont configurés et alimentés de manière similaire, une seule mesure de débit peut être suffisante. Dans ce cas, le procédé de mesure de débit décrit ci-dessus peut être employé dans un seul canal microfluidique 9. Par ailleurs, ces procédés de mesure de débit sont combinables avec les capteurs des différents exemples.

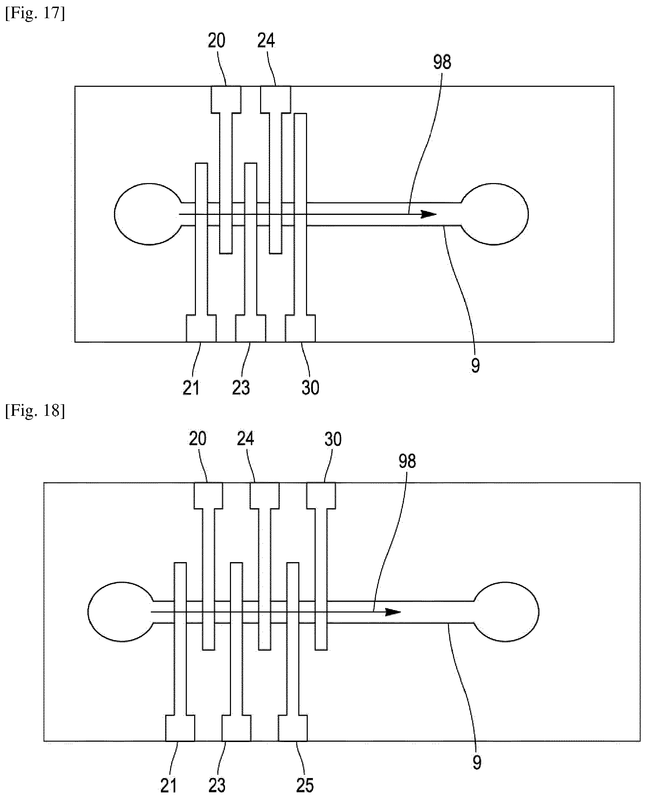

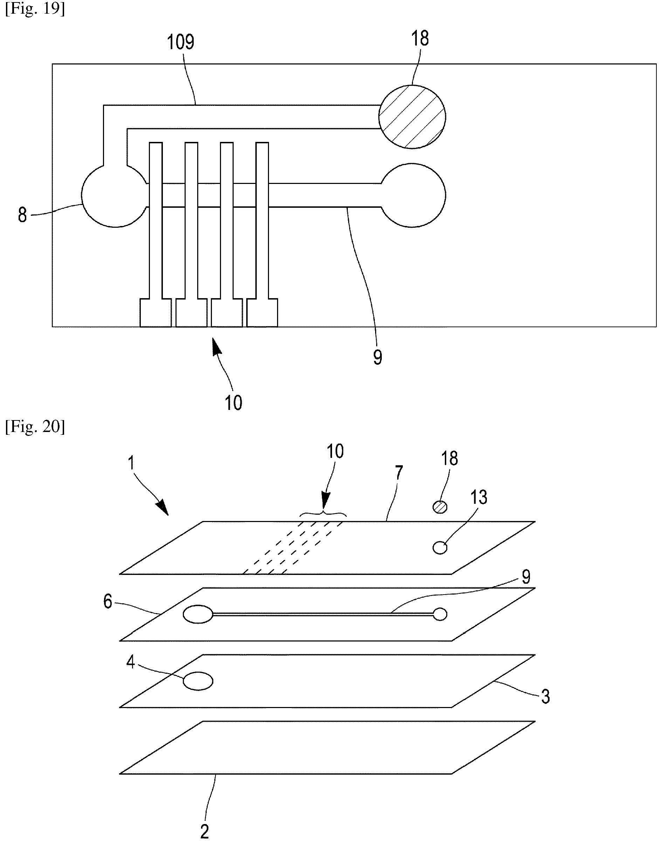

En référence aux figures 18 et 19, on décrit des capteurs électrochimiques employant un plus grand nombre d'électrodes.

Les électrodes présentées sur les figures 17 à 19 peuvent être disposées sur la face interne de la couche supérieure fermant le canal microfluidique par le haut et/ou disposées sur une face supérieure de la couche inférieure fermant le canal microfluidique par le bas. La représentation schématique ne distingue pas les différentes couches.

La [Fig.17] représente une configuration d'électrode selon un mode de réalisation dans lequel le capteur électrochimique comporte, de gauche à droite de la [Fig.17], une électrode de référence 21, une électrode de déplétion 20, une première électrode de travail 23, une deuxième électrode de travail 24 et une contre électrode 30. Cette configuration permet de réaliser dans l'ordre suivant:

une déplétion d'une espèce chimique non désirée telle que le peroxyde d'hydrogène via la polarisation de l'électrode de déplétion 20 au potentiel d'oxydation du peroxyde d'hydrogène ;
une oxydation du monoxyde d'azote via la première électrode de travail 23 dont le potentiel d'oxydation est supérieur au potentiel d'oxydation du peroxyde d'hydrogène ;
une oxydation des nitrites via la deuxième électrode de travail 24 dont le potentiel d'oxydation est supérieure au potentiel d'oxydation du peroxyde d'hydrogène et au potentiel d'oxydation du monoxyde d'azote, ainsi qu'une mesure du débit du flux de sueur par un retard entre la première électrode de travail 23 et la deuxième électrode de travail 24. La mesure du flux est également mesurable par un retard entre l'électrode de déplétion 20 et la première électrode de travail 23.

La [Fig.18] illustre un mode de réalisation similaire à la figure précédente. Il diffère en ce que le capteur électrochimique comporte une troisième électrode de travail 25 située entre la deuxième électrode de travail 24 et la contre électrode 30. Le débit du flux de sueur 98 est mesuré entre la deuxième électrode de travail 24 et la troisième électrode de travail 25. En espaçant les électrodes 24 et 25, c'est-à-dire en décalant les électrodes 25 et 30 plus loin vers l'extrémité du canal 9, on peut améliorer la résolution de la mesure de débit du flux de sueur 98.

La [Fig.19] représente une variante de réalisation dans laquelle l'appareil de détection comporte un circuit microfluidique 8 comprenant deux canaux microfluidiques 9 et 109 parallèles. Un premier canal microfluidique 9 comprend un capteur électrochimique. L'ensemble des capteurs électrochimiques divulgués ici peuvent être intégrés dans ce premier canal microfluidique 9. Le deuxième canal microfluidique 109 comprend un dispositif de détection par colorimétrie 18. Le dispositif de détection par colorimétrie 18 comprend une membrane microporeuse hydrophile. La membrane microporeuse hydrophile comprend au moins un réactif chimique capable de réagir avec une espèce chimique à détecter, par exemple : ion nitrite, peroxyde d'hydrogène, peroxynitrite, dioxyde de soufre, sulfure d'hydrogène, monoxyde d'azote, monoxyde de carbone et acide hypochloreux. A mesure que l'espèce chimique à détecter s'accumule dans le corps poreux imprégné du réactif, le réactif change de couleur et l'intensité de sa couleur spécifique augmente. L'intensité de couleur peut être détectée pour fournir une mesure quantitative de la quantité de l'espèce chimique dissout dans le flux de sueur. Par exemple, le réactif utilisé est le réactif de Griess permettant de détecter l'ion nitrite en fournissant un indicateur coloré de couleur rouge.

Selon une variante de réalisation présentée sur la [Fig.20], le dispositif de détection par colorimétrie 18 est placé à la sortie du canal microfluidique 9 en série avec un capteur électrochimique 10. Pour cela, la [Fig.20] montre une structure multicouche 1 identique à la [Fig.4]. Le dispositif de détection par colorimétrie 18 étant disposé en aval du capteur électrochimique, il reçoit une solution qui a été électrolysée par le capteur électrochimique 10. La mesure colorimétrique est cependant possible sous l'hypothèse que l'espèce à détecter n'a pas été substantiellement affectée par l'opération du capteur électrochimique 10.

Les procédés de détection de concentration et de débit décrits ci-dessus peuvent être mis en œuvre de manière automatisée à l'aide d'un dispositif électronique de contrôle 40, qui est de préférence intégré à l'appareil de détection 100.

En référence à la [Fig.21], on décrit maintenant un mode de réalisation du dispositif électronique de contrôle 40 pouvant être intégré dans l'appareil de détection 100, par exemple sous la forme d'une carte électronique.

Le ou chaque capteur électrochimique 10 est connecté à un convertisseur analogique numérique 14, qui alimente lui-même un processeur 15. Le processeur 15 est par exemple programmé pour mettre en œuvre les procédés de détection de concentration et de débit décrits ci-dessus.

Une source d'énergie 16, par exemple une batterie, alimente le dispositif électronique de contrôle 40. Un module de communication 17, filaire ou non filaire, peut aussi être prévu pour communiquer les résultats des mesures de concentration, de débit et/ou ou de flux de quantité de matière, pour une ou chaque espèce chimique visée, à un appareil de stockage ou de post-traitement.

La [Fig.22] représente un procédé pouvant être mis en œuvre par le processeur 15 dans un mode de réalisation.

A l'étape 31, la concentration instantanée $Cs(t)$ d'une espèce chimique S est déterminée à partir des mesures électrochimiques.

A l'étape 32, le débit volumique $d(t)$ dans le circuit fluidique correspondant est déterminé.

A l'étape 33, le flux de quantité de matière pour l'espèce chimique considérée est déterminé sur la base de $Cs(t)$ et $d(t)$, par exemple :

$P_s(t) = C_s(t).d(t)$

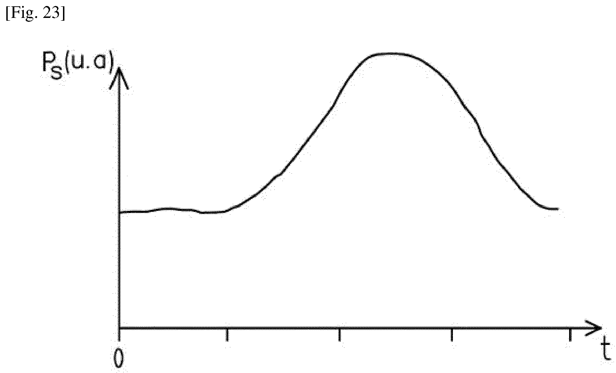

La [Fig.23] est un graphique illustrant un signal de mesure de flux de quantité de matière en fonction du temps pouvant être obtenu avec l'appareil de détection 100, par exemple au cours d'un test d'effort d'un sujet pour l'espèce NO.

**[0185]** Le dispositif électronique de contrôle 40 comporte éventuellement d'autres modules fonctionnels, par exemple un module gyroscopique et/ou accélérométrique pour détecter l'orientation et les mouvements du sujet ainsi que le niveau d'activité du sujet, et un capteur de température pour mesurer la température de l'épiderme du sujet. Il est utile de connaitre la température de la peau en raison des corrélations entre la température et la dilatation des vaisseaux.

Certains éléments de l'appareil de détection 100, notamment le dispositif électronique de contrôle 40 peuvent être réalisés sous différentes formes, de manière unitaire ou distribuée, au moyen de composants matériels et/ou logiciels. Des composants matériels utilisables sont les circuits intégrés spécifiques ASIC, les réseaux logiques programmables FPGA ou les microprocesseurs. Des composants logiciels peuvent être écrits dans différents langages de programmation, par exemple C, C++, Java ou VHDL. Cette liste n'est pas exhaustive.

Bien que l'invention ait été décrite en liaison avec plusieurs modes de réalisation particuliers, il est bien évident qu'elle n'y est nullement limitée et qu'elle comprend tous les équivalents techniques des moyens décrits ainsi que leurs combinaisons si celles-ci entrent dans le cadre de l'invention. Par exemple, les appareils de détection décrits peuvent comporter un canal microfluidique supplémentaire ou des capteurs électrochimiques différents et/ou comprenant un nombre d'électrodes différent.

L'usage du verbe « comporter », « comprendre » ou « inclure » et de ses formes conjuguées n'exclut pas la présence d'autres éléments ou d'autres étapes que ceux énoncés dans une revendication.

Dans les revendications, tout signe de référence entre parenthèses ne saurait être interprété comme une limitation de la revendication.

**Revendications**

1. Appareil de détection destiné à être posé sur une zone d'investigation (97) d'un épiderme d'un sujet humain ou animal pour détecter au moins le monoxyde d'azote dissout dans la sueur, ledit appareil de détection (100) comportant :

   une structure définissant un circuit microfluidique (8), la structure comprenant un orifice d'entrée (4) laissant passer la sueur depuis l'épiderme, le circuit microfluidique (8) comportant au moins un canal microfluidique (9) pour conduire un flux de sueur (98), le canal microfluidique (9) étant en communication avec l'orifice d'entrée (4), au moins un capteur électrochimique (10) configuré pour produire au moins un signal représentatif d'une concentration du monoxyde d'azote dissout dans le flux de sueur (98) du canal microfluidique (9), **caractérisé en ce que** le capteur électrochimique (10) comporte au moins quatre électrodes disposées de manière successive dans une direction longitudinale du canal microfluidique (9), les au moins quatre électrodes comportant une électrode de référence (21), au moins deux électrodes de travail (20, 23) et une contre-électrode (30), et **en ce que** le capteur électrochimique est configuré en outre pour faire au moins une opération supplémentaire parmi : réaliser une déplétion d'une espèce chimique dans le flux de sueur du canal microfluidique (9), ladite espèce chimique présentant un potentiel d'oxydation inférieur au potentiel d'oxydation du monoxyde d'azote et produire un signal représentatif d'une vitesse d'écoulement du flux de sueur dans le canal microfluidique (9).

2. Appareil de détection selon la revendication 1, dans lequel la structure

   est une structure multicouche (1) comportant une couche inférieure (3) et au moins une couche superposée sur la couche inférieure (3), le circuit microfluidique (8) s'étendant parallèlement à la couche inférieure (3), la couche inférieure (3) comprenant ledit orifice d'entrée (4).

3. Appareil de détection selon la revendication 2, dans lequel la structure multicouche (1) comporte une couche supérieure (7) et au moins une couche intermédiaire (6) située entre la couche inférieure (3) et la couche supérieure (7), le circuit microfluidique (8) étant formé dans l'épaisseur de la au moins une couche intermédiaire (6).

4. Appareil de détection selon la revendication 3, dans lequel la au moins une couche intermédiaire comporte une première couche intermédiaire (6) et une deuxième couche intermédiaire d'étanchéité (26) située entre la première couche intermédiaire (6) et la couche supérieure (7), la deuxième couche intermédiaire d'étanchéité (26) comportant une ouverture (28) au niveau des électrodes.

5. Appareil de détection selon l'une des revendications 2 à 4, dans lequel la structure multicouche (1) comporte une couche supérieure (7) et un orifice de sortie (13) traversant la couche supérieure (7), dans lequel le au moins un canal microfluidique (9) est en communication avec l'orifice de sortie (13).

6. Appareil de détection selon l'une des revendications 3 à 5, dans lequel les au moins quatre électrodes sont disposées sur une face interne de la couche supérieure (7) fermant le canal microfluidique (9) par le haut et/ ou sur une face supérieure de la couche inférieure (3) fermant le canal microfluidique (9) par le bas.

7. Appareil de détection selon l'une des revendications 1 à 6, dans lequel, dans la direction du flux (98), les au moins quatre électrodes comportent successivement la première électrode de travail (20) réalisée sous la forme d'une électrode de déplétion, la deuxième électrode de travail (23) pour mesurer la concentration du monoxyde d'azote et la contre-électrode, l'électrode de référence (21) étant placée à une position immédiatement en amont de la première électrode de travail (20) ou immédiatement en aval de la deuxième électrode de travail (23).

8. Appareil de détection selon l'une des revendications précédentes, dans lequel dans la direction du flux (98), les au moins quatre électrodes comportent successivement la première électrode de travail (20) pour mesurer la concentration du monoxyde d'azote, la deuxième électrode de travail (23) pour mesurer la concentration du monoxyde d'azote et la contre-électrode, l'électrode de référence (21) étant placée à une position immédiatement en amont de la première électrode de travail (20) ou immédiatement en aval de la deuxième électrode de travail (23).

9. Appareil de détection selon la revendication 7 ou 8, dans lequel le capteur électrochimique (10) est configuré pour produire le signal représentatif de la vitesse d'écoulement par mesure d'un retard ($\Delta$t) entre une variation de courant dans la première électrode de travail (20) et une variation de courant dans la deuxième électrode de travail (23).

10. Appareil de détection selon l'une des revendications précédentes, dans lequel le capteur électrochimique (10) est configuré pour produire un signal représentatif d'une production instantanée du monoxyde d'azote dans la zone d'investigation (97) sur la base du signal représentatif de la concentration du monoxyde d'azote et du signal représentatif de la vitesse d'écoulement du flux de sueur (98).

11. Appareil de détection selon l'une des revendications précédentes, dans lequel le capteur électrochimique (10) est configuré pour produire le signal représentatif de la concentration du monoxyde d'azote par une mesure électrique, notamment ampérométrique, entre au moins une desdites électrodes de travail (20, 23) et la contre-électrode (30).

12. Appareil de détection selon l'une des revendications précédentes, dans lequel le capteur électrochimique (10) est configuré pour polariser au moins une desdites électrodes de travail (20, 23) à un potentiel électrique d'oxydation du monoxyde d'azote.

13. Appareil de détection selon l'une des revendications précédentes, dans lequel le capteur électrochimique (10) est configuré pour produire un signal représentatif d'une concentration dans le flux de sueur d'au moins un des composés chimiques suivants : ion nitrite, peroxyde d'hydrogène et peroxynitrite, dissout dans la sueur.

14. Appareil de détection selon la revendication précédente, dans lequel le capteur électrochimique (10) comprend une troisième électrode de travail (25) entre la première ou deuxième électrode de travail (20, 23) et la contre électrode pour faire une mesure du composé chimique.

15. Appareil de détection selon l'une des revendications précédentes, comportant un dispositif de détection par colorimétrie (18) relié au canal (9) en aval du capteur électrochimique (10), le dispositif de détection par colorimétrie (18) comprenant un corps poreux hydrophile imprégné d'un réactif chimique capable de réagir avec un des composés chimiques suivants : ion nitrite, peroxyde d'hydrogène, peroxynitrite, dioxyde de soufre, sulfure d'hydrogène, monoxyde d'azote, monoxyde de carbone et acide hypochloreux, dissout dans la sueur, afin de fournir un indicateur coloré indiquant une quantité dudit composé chimiques dans le flux de sueur (98).

16. Appareil de détection selon la revendication 15, dans lequel le réactif chimique comprend un réactif de Griess capable de réagir avec l'ion nitrite dissout dans le flux de sueur (98).

17. Appareil de détection selon l'une des revendications 15 et 16 prise en combinaison avec la revendication 5, dans lequel le dispositif de détection par colorimétrie (18) est disposé dans l'orifice de sortie (13).

18. Appareil de détection selon l'une des revendications précédentes, dans lequel le capteur électrochimique (10) est configuré pour polariser au moins une dite électrode de travail (20, 23, 25) pendant une durée déterminée avec une récurrence périodique.

19. Appareil de détection selon l'une des revendications précédentes, dans lequel le circuit microfluidique (8) comporte une pluralité de canaux microfluidiques (9) conduisant chacun un flux de sueur, reliés en dérivation les uns des autres à l'orifice d'entrée (4).

20. Appareil de détection selon la revendication précédente, dans lequel la pluralité de canaux microfluidiques (9) comporte un canal microfluidique (9) supplémentaire comportant un capteur électrochimique (10), le capteur électrochimique (10) comportant au moins trois électrodes disposées de manière successive dans une direction longitudinale du canal microfluidique (9) supplémentaire, les au moins trois électrodes comportant une électrode de référence (21), une contre-électrode (30) et au moins une électrode de travail (20, 23, 25, le capteur électrochimique (10) supplémentaire étant configuré pour polariser les électrodes à un potentiel électrique d'oxydation d'un composé chimique choisi parmi ion nitrite, peroxyde d'hydrogène et peroxynitrite et étant configuré pour produire au moins un signal représentatif d'une concentration dudit composé chimique dissout dans un flux de sueur du canal microfluidique (9) supplémentaire.

21. Appareil de détection selon l'une des revendications 19 à 20, dans lequel la pluralité de canaux microfluidiques comporte un canal microfluidique (109) supplémentaire comportant un dispositif de détection par colorimétrie (18), le dispositif de détection par colorimétrie (18) comprenant un corps poreux hydrophile imprégné d'un réactif chimique capable de réagir avec un des composés chimiques suivants : ion nitrite, peroxyde d'hydrogène, peroxynitrite, dioxyde de soufre, sulfure d'hydrogène, monoxyde d'azote, monoxyde de carbone et acide hypochloreux, afin de fournir un indicateur coloré indiquant une concentration ou une quantité du composé chimique dissout dans un flux de sueur du canal microfluidique (109) supplémentaire .

22. Appareil de détection selon la revendication 21, dans lequel le canal supplémentaire (109) comporte un système de chrono-échantillonnage relié à l'orifice d'entrée (4), le système de chrono-échantillonnage incluant une pluralité de chambres configurées pour se remplir de manière séquentielle de la sueur, et dans lequel une pluralité de dispositifs de détection par colorimétrie (18) sont disposés dans lesdites chambres, chaque dispositif de détection par colorimétrie (18) comprenant un réactif chimique capable de réagir avec un composé chimique, de sorte que les dispositifs de détection par colorimétrie disposés dans lesdites chambres fournissent un indicateur coloré indiquant une quantité cumulée dudit composé chimique dans le flux de sueur du canal microfluidique (109) supplémentaire .

23. Appareil de détection selon l'une des revendications 15 à 17 et 21 à 22, comportant un capteur optique configuré pour produire un signal de mesure représentatif de l'intensité d'une couleur du réactif chimique dans le spectre visible ou ultraviolet.

24. Appareil de détection selon l'une des revendications précédentes, comportant un dispositif de communication (17) configuré pour transmettre un ou plusieurs signaux de mesure produits par l'appareil de détection (100) à destination d'un appareil de stockage ou de post-traitement.

25. Dispositif portatif comprenant un appareil de détection (100) selon l'une des revendications 1 à 24, le dispositif portatif étant réalisé sous la forme de : une montre, un téléphone, un tissu, un bandeau, un vêtement, ou un sous vêtement.

**Patentansprüche**

1. Detektionsvorrichtung zur Aufbringung auf einem Untersuchungsbereich (97) der Epidermis eines menschlichen oder tierischen Subjekts, um zumindest das im Schweiß gelöste Stickstoffmonoxid zu detektieren, wobei die Detektionsvorrichtung (100) umfasst:

eine Struktur, die eine mikrofluidische Schaltung (8) definiert, wobei die Struktur eine Einlassöffnung (4) umfasst, die Schweiß von der Epidermis durchlässt, wobei die mikrofluidische Schaltung (8) mindestens einen mikrofluidischen Kanal (9) zum Leiten eines Schweißstroms (98) umfasst, wobei der mikrofluidische Kanal (9) in Verbindung mit der Einlassöffnung (4) steht,

mindestens einen elektrochemischen Sensor (10), der so konfiguriert ist, dass dieser mindestens ein Signal erzeugt, das repräsentativ für eine Konzentration von Stickstoffmonoxid ist, das in dem Schweißstrom (98) des mikrofluidischen Kanals (9) gelöst ist,

**dadurch gekennzeichnet, dass** der elektrochemische Sensor (10) mindestens vier Elektroden aufweist, die in einer Längsrichtung des mikrofluidischen Kanals (9) aufeinanderfolgend angeordnet sind, wobei die mindestens vier Elektroden eine Referenzelektrode (21), mindestens zwei Arbeitselektroden (20, 23) und eine Gegenelektrode (30) umfassen,

und dass der elektrochemische Sensor ferner so konfiguriert ist, dass dieser mindestens eine der folgenden zusätzlichen Operationen ausführt:

Durchführung einer Depletion einer chemischen Substanz im Schweißstrom des mikrofluidischen Kanals (9), wobei die chemische Substanz ein Oxidationspotential aufweist, das geringer ist als das Oxidationspotential von Stickstoffmonoxid, und

Erzeugung eines Signals, das für eine Fließgeschwindigkeit des Schweißstroms im mikrofluidischen Kanal (9) repräsentativ ist.

2. Detektionsvorrichtung nach Anspruch 1, wobei die Struktur eine mehrschichtige Struktur (1) umfassend eine untere Schicht (3) und mindestens eine über der unteren Schicht (3) liegenden Schicht, wobei sich die mikrofluidische Schaltung (8) parallel zur unteren Schicht (3) erstreckt, wobei die untere Schicht (3) die Einlassöffnung (4) umfasst.

3. Detektionsvorrichtung nach Anspruch 2, wobei die mehrschichtige Struktur ( 1) eine obere Schicht (7) und mindestens eine Zwischenschicht (6) aufweist, die zwischen der unteren Schicht (3) und der oberen Schicht (7) angeordnet ist, wobei die mikrofluidische Schaltung (8) in der Dicke der mindestens einen Zwischenschicht (6) ausgebildet ist.

4. Detektionsvorrichtung nach Anspruch 3, wobei die mindestens eine Zwischenschicht eine erste Zwischenschicht (6) und eine zweite Dichtungszwischenschicht (26) umfasst, die zwischen der ersten Zwischenschicht (6) und der oberen Schicht (7) anordnet ist, wobei die zweite Dichtungszwischenschicht (26) eine Öffnung (28) an den Elektroden aufweist.

5. Detektionsvorrichtung nach einem der Ansprüche 2 bis 4, wobei die mehrschichtige Struktur (1) eine obere Schicht (7) und eine Austrittsöffnung (13) aufweist, die durch die obere Schicht (7) verläuft, wobei der mindestens eine mikrofluidische Kanal (9) mit der Austrittsöffnung (13) in Verbindung steht.

6. Detektionsvorrichtung nach einem der Ansprüche 3 bis 5, wobei die mindestens vier Elektroden auf einer Innenseite der oberen Schicht (7), die den mikrofluidischen Kanal (9) von oben verschließt, und/oder auf einer Oberseite der unteren Schicht (3), die den mikrofluidischen Kanal (9) von unten verschließt, angeordnet sind.

7. Detektionsvorrichtung nach einem der Ansprüche 1 bis 6, wobei in der Richtung des Stroms (98) die mindestens vier Elektroden nacheinander die erste Arbeitselektrode (20), die als Depletionselektrode ausgebildet ist, die zweite Arbeitselektrode (23) zur Messung der Stickstoffmonoxidkonzentration und die Gegenelektrode umfassen, wobei die Referenzelektrode (21) an einer Stelle unmittelbar vor der ersten Arbeitselektrode (20) oder unmittelbar hinter der zweiten Arbeitselektrode (23) angeordnet ist.

8. Detektionsvorrichtung nach einem der vorhergehenden Ansprüche, wobei in der Richtung des Stroms (98) die mindestens vier Elektroden nacheinander die erste Arbeitselektrode (20) zum Messen der Stickstoffmonoxidkonzentration, die zweite Arbeitselektrode (23) zum Messen der Stickstoffmonoxidkonzentration und die Gegenelektrode umfassen, wobei die Referenzelektrode (21) an einer Position unmittelbar vor der ersten Arbeitselektrode (20) oder unmittelbar hinter der zweiten Arbeitselektrode (23) angeordnet ist.

9. Detektionsvorrichtung nach Anspruch 7 oder 8, wobei der elektrochemische Sensor (10) so konfiguriert ist, dass dieser das Signal, das die Fließgeschwindigkeit repräsentiert, durch Messung einer Verzögerungszeit ($\Delta t$) zwischen einer Stromänderung in der ersten Arbeitselektrode (20) und einer Stromänderung in der zweiten Arbeitselektrode (23) erzeugt.

10. Detektionsvorrichtung nach einem der vorhergehenden Ansprüche, wobei der elektrochemische Sensor (10) so konfiguriert ist, dass dieser ein Signal erzeugt, das eine momentane Stickstoffmonoxidproduktion in dem Untersuchungsbereich (97) auf der Grundlage des Signals, das die Stickstoffmonoxidkonzentration repräsentiert, und des Signals, das die Fließgeschwindigkeit des Schweißflusses (98) repräsentiert, repräsentiert.

**11.** Detektionsvorrichtung nach einem der vorhergehenden Ansprüche, wobei der elektrochemische Sensor (10) so konfiguriert ist, dass dieser das Signal, das die Konzentration von Stickstoffmonoxid repräsentiert, durch eine elektrische, insbesondere amperometrische, Messung zwischen mindestens einer der Arbeitselektroden (20, 23) und der Gegenelektrode (30) erzeugt.

**12.** Detektionsvorrichtung nach einem der vorhergehenden Ansprüche, wobei der elektrochemische Sensor (10) so konfiguriert ist, dass dieser mindestens eine der Arbeitselektroden (20, 23) auf ein elektrisches Stickstoffmonoxid-Oxidationspotential polarisiert.

**13.** Detektionsvorrichtung nach einem der vorhergehenden Ansprüche, wobei der elektrochemische Sensor (10) so konfiguriert ist, dass dieser ein Signal erzeugt, das eine Konzentration im Schweißstrom von mindestens einer der folgenden chemischen Verbindungen darstellt: Nitrit-Ion, Wasserstoffperoxid und Peroxynitrit, die in dem Schweiß gelöst sind.

**14.** Detektionsvorrichtung nach dem vorhergehenden Anspruch, wobei der elektrochemische Sensor (10) eine dritte Arbeitselektrode (25) zwischen der ersten oder zweiten Arbeitselektrode (20, 23) und der Gegenelektrode umfasst, um eine Messung der chemischen Verbindung vorzunehmen.

**15.** Detektionsvorrichtung nach einem der vorhergehenden Ansprüche, umfassend eine kolorimetrischen Detektionseinrichtung (18), die mit dem Kanal (9) stromabwärts des elektrochemischen Sensors (10) verbunden ist, wobei die kolorimetrische Detektionseinrichtung (18) einen hydrophilen porösen Körper umfasst, der mit einem chemischen Reagenz imprägniert ist, das mit einer der folgenden chemischen Verbindungen reagieren kann: Nitrit-Ion, Wasserstoffperoxid, Peroxynitrit, Schwefeldioxid, Schwefelwasserstoff, Stickstoffmonoxid, Kohlenmonoxid und hypochlorige Säure, die im Schweiß gelöst sind, um einen Farbindikator zu bereitzustellen, der eine Menge der genannten chemischen Verbindung im Schweißstrom (98) anzeigt.

**16.** Detektionsvorrichtung nach Anspruch 15, wobei das chemische Reagenz ein Griess-Reagenz umfasst, das mit dem im Schweißstrom (98) gelösten Nitrition reagieren kann.

**17.** Detektionsvorrichtung nach einem der Ansprüche 15 und 16 in Kombination mit Anspruch 5, wobei die kolorimetrische Detektionsvorrichtung (18) in der Austrittsöffnung (13) angeordnet ist.

**18.** Detektionsvorrichtung nach einem der vorhergehenden Ansprüche, wobei der elektrochemische Sensor (10) so konfiguriert ist, dass dieser mindestens eine der Arbeitselektroden (20, 23, 25) für eine bestimmte Zeitdauer in regelmäßigr Wiederholung polarisiert.

**19.** Detektionsvorrichtung nach einem der vorhergehenden Ansprüche, bei der die Mikrofluidische Schaltung (8) eine Vielzahl von mikrofluidischen Kanäle (9) aufweist, die jeweils einen Schweißstrom leiten und in Abzweigung voneinander mit der Einlassöffnung (4) verbunden sind.

**20.** Detektionsvorrichtung nach dem vorhergehenden Anspruch, wobei die Vielzahl von mikrofluidischen Kanälen (9) einen weiteren mikrofluidischen Kanal (9) mit einem elektrochemischen Sensor (10) umfasst, wobei der elektrochemische Sensor (10) mindestens drei Elektroden umfasst, die aufeinanderfolgend in einer Längsrichtung des weiteren mikrofluidischen Kanals (9) angeordnet sind, wobei die mindestens drei Elektroden eine Referenzelektrode (21), eine Gegenelektrode (30) und mindestens eine Arbeitselektrode (20, 23, 25) umfassen, wobei der weitere elektrochemische Sensor (10) so konfiguriert ist, dass dieser die Elektroden auf ein elektrisches Oxidationspotential einer chemischen Verbindung polarisiert, die aus Nitrit-Ion, Wasserstoffperoxid und Peroxynitrit ausgewählt ist, und so konfiguriert ist, dass dieser mindestens ein Signal erzeugt, das für eine Konzentration der chemischen Verbindung repräsentativ ist, die in einem Schweißstrom aus dem weiteren mikrofluidischen Kanal (9) gelöst ist.

**21.** Detektionsvorrichtung nach einem der Ansprüche 19 bis 20, wobei die Vielzahl von Mikrofluidkanälen einen weiteren Mikrofluidkanal (109) mit einer kolorimetrischen Detektionseinrichtung (18) aufweist, wobei die kolorimetrische Detektionseinrichtung (18) einen hydrophilen porösen Körper umfasst, der mit einem chemischen Reagenz imprägniert ist, das mit einer der folgenden chemischen Verbindungen reagieren kann: Nitrit-Ion, Wasserstoffperoxid, Peroxynitrit, Schwefeldioxid, Schwefelwasserstoff, Stickstoffmonoxid, Kohlenmonoxid und hypochlorige Säure, um einen Farbindikator bereitzustellen, der eine Konzentration oder Menge der in einem Schweißstrom des weiteren mikrofluidischen Kanals (109) gelösten chemischen Verbindung anzeigt.

22. Detektionsvorrichtung nach Anspruch 21, wobei der weitere Kanal (109) ein mit der Einlassöffnung (4) verbundenes Chrono-Sampling-System aufweist, wobei das Chrono-Sampling-System eine Vielzahl von Kammern umfasst, die so konfiguriert sind, dass diese sich sequentiell mit Schweiß füllen, und wobei eine Vielzahl von kolorimetrischen Detektionsvorrichtungen (18) in den Kammern angeordnet sind, wobei jede kolorimetrische Detektionsvorrichtung (18) ein chemisches Reagenz umfasst, das mit einer chemischen Verbindung reagieren kann, so dass die in den Kammern angeordneten kolorimetrischen Detektionsvorrichtungen einen Farbindikator bereitstellen, der eine kumulierte Menge der chemischen Verbindung im Schweißstrom des weiteren mikrofluidischen Kanals (109) anzeigt.

23. Detektionsvorrichtung nach einem der Anspruche 15 bis 17 und 21 bis 22, umfassend einen optischen Sensor, der so konfiguriert ist, dass dieser ein Messsignal erzeugt, das für die Intensität einer Farbe des chemischen Reagens im sichtbaren oder ultravioletten Spektrum repräsentativ ist.

24. Detektionsvorrichtung nach einem der vorhergehenden Ansprüche umfassend eine Kommunikationsvorrichtung (17), die so konfiguriert ist, dass diese ein oder mehrere von der Detektionsvorrichtung (100) erzeugte Messsignale an ein Speicher- oder Nachverarbeitungsgerät überträgt.

25. Tragbare Vorrichtung umfassend eine Detektionsvorrichtung (100) nach einem der Ansprüche 1 bis 24, wobei die tragbare Vorrichtung in Form einer Uhr, eines Telefons, eines Stoffes, eines Stirnbandes, einer Kleidung oder einer Unterkleidung ausgebildet ist.

**Claims**

1. A detection apparatus for placement on an investigation zone (97) of an epidermis of a human or animal subject for detecting at least the nitric oxide dissolved in sweat, said detection apparatus (100) comprising:

   a structure defining a microfluidic circuit (8), the structure comprising an entry orifice (4) allowing passage of sweat from the epidermis, the microfluidic circuit (8) comprising at least one microfluidic channel (9) for guiding a flow of sweat (98), the microfluidic channel (9) being in communication with the entry orifice (4),
   at least one electrochemical sensor (10) configured to produce at least one signal that is representative of a concentration of the nitric oxide dissolved in the flow of sweat (98) in the microfluidic channel (9),
   **characterized in that** the electrochemical sensor (10) comprises at least four electrodes disposed successively in a longitudinal direction of the microfluidic channel (9), the at least four electrodes comprising a reference electrode (21), at least two working electrodes (20, 23) and a counter-electrode (30),
   and **in that** the electrochemical sensor is further configured to perform at least one additional operation from among the following:

   depleting a chemical species in the flow of sweat in the microfluidic channel (9), said chemical species having an oxidation potential lower than the oxidation potential of nitric oxide, and
   producing a signal that is representative of a flow rate of the flow of sweat in the microfluidic channel (9).

2. The detection apparatus as claimed in claim 1, in which the structure is a multilayer structure (1) comprising a lower layer (3) and at least one layer atop the lower layer (3), the microfluidic circuit (8) extending parallel to the lower layer (3), and the lower layer (3) comprising said entry orifice (4).

3. The detection apparatus as claimed in claim 2, in which the multilayer structure (1) comprises an upper layer (7) and at least one middle layer (6) situated between the lower layer (3) and the upper layer (7), the microfluidic circuit (8) being formed in the thickness of the at least one middle layer (6).

4. The detection apparatus as claimed in claim 3, in which the at least one middle layer comprises a first middle layer (6) and a second, sealing middle layer (26) situated between the first middle layer (6) and the upper layer (7), the second, sealing middle layer (26) comprising an opening (28) at the electrodes.

5. The detection apparatus as claimed in any of claims 2 to 4, in which the multilayer structure (1) comprises an upper layer (7) and an outlet orifice (13) traversing the upper layer (7), in which the at least one microfluidic channel (9) is in communication with the outlet orifice (13).

6. The detection apparatus as claimed in any of claims 3 to 5, in which the at least four electrodes are disposed on an inner face of the upper layer (7) closing the microfluidic channel (9) at the top and/or on an upper face of the lower layer (3) closing the microfluidic channel (9) at the bottom.

7. The detection apparatus as claimed in any of claims 1 to 6, in which, in the direction of the flow (98), the at least four electrodes comprise in succession the first working electrode (20), in the form of a depletion electrode, the second working electrode (23) for measuring the concentration of nitric oxide, and the counter-electrode, the reference electrode (21) being placed at a position immediately upstream of the first working electrode (20) or immediately downstream of the second working electrode (23).

8. The detection apparatus as claimed in any of the preceding claims, in which, in the direction of the flow (98), the at least four electrodes comprise in succession the first working electrode (20) for measuring the concentration of nitric oxide, the second working electrode (23) for measuring the concentration of nitric oxide, and the counter-electrode, the reference electrode (21) being placed at a position immediately upstream of the first working electrode (20) or immediately downstream of the second working electrode (23).

9. The detection apparatus as claimed in claim 7 or 8, in which the electrochemical sensor (10) is configured to produce the signal that is representative of the flow rate by measuring a delay ($\Delta t$) between a variation in current in the first working electrode (20) and a variation in current in the second working electrode (23).

10. The detection apparatus as claimed in any of the preceding claims, in which the electrochemical sensor (10) is configured to produce a signal that is representative of instantaneous production of nitric oxide in the investigation zone (97) on the basis of the signal that is representative of the concentration of nitric oxide and of the signal that is representative of the flow rate of the flow of sweat (98).

11. The detection apparatus as claimed in any of the preceding claims, in which the electrochemical sensor (10) is configured to produce the signal that is representative of the concentration of nitric oxide by an electrical, especially amperometric, measurement between at least one of said working electrodes (20, 23) and the counter-electrode (30).

12. The detection apparatus as claimed in any of the preceding claims, in which the electrochemical sensor (10) is configured to polarize at least one of said working electrodes (20, 23) to an electrical potential for oxidation of nitric oxide.

13. The detection apparatus as claimed in any of the preceding claims, in which the electrochemical sensor (10) is configured to produce a signal that is representative of a concentration in the flow of sweat of at least one of the following chemical compounds: nitrite ion, hydrogen peroxide and peroxynitrite, dissolved in sweat.

14. The detection apparatus as claimed in the preceding claim, in which the electrochemical sensor (10) comprises a third working electrode (25) between the first or second working electrode (20, 23) and the counter-electrode for measuring the chemical compound.

15. The detection apparatus as claimed in any of the preceding claims, comprising a colorimetric detection device (18) connected to the channel (9) downstream of the electrochemical sensor (10), the colorimetric detection device (18) comprising a hydrophilic porous body impregnated with a chemical reagent capable of reacting with one of the following chemical compounds: nitrite ion, hydrogen peroxide, peroxynitrite, sulfur dioxide, hydrogen sulfide, nitric oxide, carbon monoxide and hypochlorous acid, dissolved in sweat, so as to provide a colored indicator indicating a quantity of said chemical compound in the flow of sweat (98).

16. The detection apparatus as claimed in claim 15, in which the chemical reagent comprises a Griess reagent capable of reacting with the nitrite ion dissolved in the flow of sweat (98).

17. The detection apparatus as claimed in either of claims 15 and 16 taken in combination with claim 5, in which the colorimetric detection device (18) is disposed in the outlet orifice (13).

18. The detection apparatus as claimed in any of the preceding claims, in which the electrochemical sensor (10) is configured to polarize at least one said working electrode (20, 23, 25) during a determined time with a periodic recurrence.

19. The detection apparatus as claimed in any of the preceding claims, in which the microfluidic circuit (8) comprises a plurality of microfluidic channels (9) each guiding a flow of sweat, which are connected in derivation from one another to the entry orifice (4).

20. The detection apparatus as claimed in the preceding claim, in which the plurality of microfluidic channels (9) comprises an additional microfluidic channel (9) comprising an electrochemical sensor (10), the electrochemical sensor (10) comprising at least three electrodes disposed successively in a longitudinal direction of the additional microfluidic channel (9), the at least three electrodes comprising a reference electrode (21), a counter-electrode (30) and at least one working electrode (20, 23, 25), the additional electrochemical sensor (10) being configured to polarize the electrodes to an electrical potential for oxidation of a chemical compound selected from nitrite ion, hydrogen peroxide and peroxynitrite and being configured to produce at least one signal that is representative of a concentration of said chemical compound dissolved in a flow of sweat in the additional microfluidic channel (9).

21. The detection apparatus as claimed in either of claims 19 to 20, in which the plurality of microfluidic channels comprises an additional microfluidic channel (109) comprising a colorimetric detection device (18), the colorimetric detection device (18) comprising a hydrophilic porous body impregnated with a chemical reagent capable of reacting with one of the following chemical compounds: nitrite ion, hydrogen peroxide, peroxynitrite, sulfur dioxide, hydrogen sulfide, nitric oxide, carbon monoxide and hypochlorous acid, so as to provide a colored indicator indicating a concentration or a quantity of the chemical compound dissolved in a flow of sweat in the additional microfluidic channel (109).

22. The detection apparatus as claimed in claim 21, in which the additional channel (109) comprises a chrono-sampling system connected to the entry orifice (4), the chrono-sampling system including a plurality of chambers configured to fill sequentially with sweat, and in which a plurality of colorimetric detection devices (18) are disposed in said chambers, each colorimetric detection device (18) comprising a chemical reagent capable of reacting with a chemical compound, such that the colorimetric detection devices disposed in said chambers provide a colored indicator indicating a cumulative quantity of said chemical compound in the flow of sweat in the additional microfluidic channel (109).

23. The detection apparatus as claimed in any of claims 15 to 17 and 21 to 22, comprising an optical sensor configured to produce a measurement signal that is representative of the intensity of a color of the chemical reagent in the visible or ultraviolet spectrum.

24. The detection apparatus as claimed in any of the preceding claims, comprising a communication device (17) configured to transmit one or more measurement signals produced by the detection apparatus (100) to a storage or post-processing apparatus.

25. A portable device comprising a detection apparatus (100) as claimed in any of claims 1 to 24, the portable device being implemented in the form of: a watch, a telephone, a fabric, a headband, a garment or an undergarment.

[Fig. 1]

[Fig. 2]

[Fig. 3]

[Fig. 4]

[Fig. 5]

[Fig. 6]

[Fig. 7]

[Fig. 8]

[Fig. 9]

[Fig. 10]

[Fig. 11]

[Fig. 12]

[Fig. 13]

[Fig. 14]

[Fig. 15]

[Fig. 16]

[Fig. 17]

[Fig. 18]

[Fig. 19]

[Fig. 20]

[Fig. 21]

[Fig. 22]

[Fig. 23]

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2019229380 A1
- US 2019110722 A1
- WO 2016061362 A2

**Littérature non-brevet citée dans la description**

- **KENNY WALTER**. *Research & Development World*, 26 April 2019
- **CHOI et al.** *Thin, Soft, Skin-Mounted Microfluidic Networks with Capillary Bursting Valves for Chrono-Sampling of Sweat Adv. Healthcare Mater*, 2017